(19) Europäisches Patentamt
European Patent Office
Office européen des brevets

(11) **EP 1 797 822 A1**

(12) **EUROPÄISCHE PATENTANMELDUNG**

(43) Veröffentlichungstag:
**20.06.2007 Patentblatt 2007/25**

(51) Int Cl.:
*A61B 5/15* (2006.01)    *A61B 10/00* (2006.01)

(21) Anmeldenummer: **05027428.1**

(22) Anmeldetag: **15.12.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder:
• **Roche Diagnostics GmbH**
 **68305 Mannheim (DE)**
 Benannte Vertragsstaaten:
 **DE**
• **F. HOFFMANN-LA ROCHE AG**
 **4070 Basel (CH)**
 Benannte Vertragsstaaten:
 **AT BE BG CH CY CZ DK EE ES FI FR GB GR HU IE IS IT LI LT LU LV MC NL PL PT RO SE SI SK TR**

(72) Erfinder:
• **List, Hans**
 **64754 Hesseneck-Kailbach (DE)**
• **Haar, Hans-Peter**
 **69168 Wiesloch (DE)**
• **Meacham, George Bevan Kirby**
 **Ohio 44122 (US)**
• **Eusemann, Michael**
 **64354 Reinheim (DE)**

(74) Vertreter: **Pfeifer, Hans-Peter et al**
**Durm & Partner**
**Patentanwälte**
**Beiertheimer Allee 19**
**76137 Karlsruhe (DE)**

(54) **Stechsystem zur Entnahme einer Körperflüssigkeit**

(57)    Die Erfindung betrifft ein Mikrosampler-Stechsystem zur Entnahme einer Körperflüssigkeitsprobe aus einem Körperteil, umfassend eine Probenentnahmeeinheit mit einem Stechelement, und ein Stechgerät mit einem Antrieb, mittels dem eine Probenentnahmeeinheit auf einem Bewegungsweg beweglich ist, um das Stechelement mit einer Einstich- und Rückführbewegung in die Haut des Körperteils einzustechen und wieder herauszuziehen, einer Einstelleinrichtung zum Einstellen der Einstechtiefe der zu erzeugenden Einstichwunde, und einer Steuereinrichtung zum Steuern der Einstich- und Rückführbewegung mit folgenden nacheinander ablaufenden Bewegungsphasen: Einer Vortriebsphase (V), bei der das Stechelement (5) bis zu einer vorgegebenen Einstechtiefe (dm) eingestochen wird, einer Rückzugsphase (R1), bei der das Stechelement (5) um eine Rückzugsstrecke (Δd) teilweise zurückgezogen und gegen Ende der Rückzugsphase (R1) abgebremst wird, so daß es mit einer vorgegebenen Reststechtiefe (dr) in die Haut hineinragt und einer Sammelphase (S), in der das Stechelement in die Haut hineinragt und eine Körperflüssigkeitsprobe von der Probenaufnahmeeinheit aufgenommen wird. Erfindungsgemäß ist vorgesehen, daß die Einstelleinrichtung derart ausgebildet ist, daß die Einstechtiefe unabhängig von dem zeitlichen Mittel der sich während der Sammelphase (S) ergebenden Reststechtiefe (dr) einstellbar ist.

Fig. 1

EP 1 797 822 A1

## Beschreibung

Die Erfindung betrifft ein Mikrosampler-Stechsystem zur Entnahme einer Körperflüssigkeitsprobe aus einem Körperteil. Stechsysteme umfassen eine Probenentnahmeeinheit mit einem Stechelement sowie ein Stechgerät mit einem Antrieb, mittels dem eine Probenentnahmeeinheit auf einem Bewegungsweg beweglich ist, um das Stechelement mit einer Einführ- und Rückführbewegung in die Haut eines Körperteils einzustechen und wieder herauszuziehen.

Die Probenentnahmeeinheit eines Mikrosampler-Stechsystems dient nicht nur zum Erzeugen einer Einstichwünde, sondern zugleich auch zur Entnahme einer einer kleinen Körperflüssigkeitsprobe. Typischerweise beträgt das entnommene Probenvolumen höchstens einige Mikroliter, weshalb derartige Stechsysteme als "Mikrosampler-Stechsysteme" bezeichnet werden. Eine Probenentnahmeeinheit für ein Mikrosampler-Stechsystem ist aus der WO 03/009759 A1 bekannt. Zur Probenentnahme kann das Stechelement nach einem ersten Einstich, mit dem ein Einstichkanal erzeugt wird, bei einem zweiten Einstich erneut mit einer geringeren Tiefe in den Einstichkanal gestochen werden. Das Stechelement verbleibt nach dem zweiten Einstich während einer Sammelphase in dem Einstichkanal, so daß Körperflüssigkeit durch den Kapillarkanal aufgenommen werden kann. Eine weitere Möglichkeit der Probenentnahme besteht darin, das Stechelement nach dem Einstich zum Erzeugen des Einstichkanals nur teilweise zurückzuziehen, so daß es ebenfalls während einer Sammelphase mit einer geringeren Tiefe in dem Einstichkanal verbleibt.

Mikrosampler-Stechsysteme bedeuten für Diabetiker, die mehrmals täglich ihren Blutzuckerspiegel überprüfen müssen, durch die automatische Probenentnahme eine große Erleichterung. Aufgabe der Erfindung ist es, einen Weg aufzuzeigen, wie Mikrosampler-Stechsysteme hinsichtlich Probengewinnung und Schmerzempfinden weiter verbessert werden können.

Diese Aufgabe wird gelöst durch ein Mikrosampler-Stechsystem zur Entnahme einer Körperflüssigkeitsprobe aus einem Körperteil, umfassend eine Probenentnahmeeinheit mit einem Stechelement und ein Stechgerät mit einem Antrieb, mittels dem eine Probenentnahmeeinheit auf einem Bewegungsweg beweglich ist; um das Stechelement mit einer Einstich- und Rückführbewegung in die Haut des Körperteils einzustechen und wieder herauszuziehen, einer Einstelleinrichtung zum Einstellen der Einstechtiefe der zu erzeugenden Einstichwunde, und einer Steuereinrichtung zum Steuern der Einstich- und Rückführbewegung mit folgenden nacheinander ablaufenden Bewegungsphasen:

- einer Vortriebsphase, bei der das Stechelement bis zu einer vorgegebenen Einstechtiefe eingestochen wird,

- einer Rückzugsphase, bei der das Stechelement um eine Rückzugsstrecke teilweise zurückgezogen und gegen Ende der Rückzugsphase abgebremst wird, so daß es mit einer vorgegebenen Reststechtiefe in die Haut hineinragt und

- einer Sammelphase, in der eine Körperflüssigkeitsprobe von der Probenaufnahmeeinheit aufgenommen wird, während das Stechelement in die Haut hineinragt,

dadurch gekennzeichnet, daß die Einstelleinrichtung derart ausgebildet ist, daß die Einstechtiefe unabhängig von dem zeitlichen Mittel der sich während der Sammelphase ergebenden Reststechtiefe einstellbar ist.

Im Rahmen der Erfindung wurde festgestellt, daß für die Probengewinnung und das Schmerzempfinden die Reststechtiefe des Stechelements während der Sammelphase von gro-ßer Bedeutung ist. Dabei ist es nicht unbedingt erforderlich, daß die Reststechtiefe während der gesamten Sammelphase konstant ist. Geringe Schwankungen der Reststechtiefe oder eine langsame Bewegung des Stechelements während der Sammelphase, beispielsweise weniger als 0,3 mm/sec., bevorzugt weniger als 0,1 mm/sec., scheinen von untergeordneter Bedeutung zu sein.

Jedenfalls ist es für eine schmerzarme und effiziente Probenentnahme wichtig, daß sich die Einstechtiefe unabhängig von der Reststechtiefe einstellen läßt. Wenn die Reststechtiefe nicht zeitlich konstant ist, bezieht sich diese Aussage auf deren zeitliches Mittel während der Sammelphase. Bei vorbekannten Mikrosampler-Stechsystemen war die Konstruktion hingegen stets so ausgebildet, daß eine Veränderung der eingestellten Einstechtiefe auch zu einer für das Schmerzempfinden während der Sammelphase erheblichen Änderung der Reststechtiefe führte.

Im Rahmen der Anmeldung wird unter dem zeitlichen Mittel der Reststechtiefe <dr> der Quotient aus dem Zeitintegral der Stechtiefe d(t) von dem Beginn der Sammelphase zur Zeit t1 bis zu dem Ende der Sammelphase zur Zeit t2 und der Dauer der Sammelphase t2 minus t1 verstanden:

$$<\text{dr}> = \frac{\int_{t_1}^{t_2} d(t)dt}{t_2 - t_1}$$

Im allgemeinen werden Beginn und Ende der Sammelphase, also die Zeitpunkte t1 und t2, dadurch definiert, daß nach dem Abbremsen des Stechelements die Aufnahme der Körperflüssigkeit beginnt bzw. beendet wird. Bevorzugt wird das Stechelement am Ende der Rückzugsphase abgestoppt, so daß die Reststechtiefe während der Sammelphase konstant ist.

Ein weiterer Aspekt der Erfindung, der auch selbstständig Bedeutung hat, betrifft ein Stechgerät, insbesondere für ein Stechsystem nach einem der vorhergehenden Ansprüche, umfassend einen Lanzettenträger zur Aufnahme einer Probenentnahmeeinheit und einen Antrieb zum Beschleunigen des Lanzettenträgers für eine Einstich- und Rückführbewegung, dadurch gekennzeichnet, daß der Lanzettenträger mit einem Dämpfungsmechanismus gekoppelt ist, um das Abbremsen des Lanzettenträgers gegen Ende einer Rückführbewegung zu dämpfen.

Mit einem erfindungsgemäßen Dämpfungsmechanismus kann der Antriebsrotor am Ende einer Rückführbewegung langsam abgestoppt werden, so daß schmerzhafte Schwingungen des Stechelements zuverlässig vermieden werden können. Dies ist besonders bedeutsam, wenn das Stechelement beim Abstoppen mit einer Reststechtiefe in die Haut eines Patienten hineinragt.

Die Erfindung wird nachfolgend anhand von Ausführungsbeispielen unter Bezugnahme auf die beigefügten Zeichnungen näher erläutert. Die darin dargestellten Besonderheiten können einzeln oder in Kombination verwendet werden, um bevorzugte Ausgestaltungen zu schaffen. Es zeigen:

Fig. 1     ein Beispiel des zeitlichen Verlaufs der Stechtiefe;

Fig. 2     eine Prinzipskizze eines Beispiels eines erfindungsgemäßen Stechgeräts;

Fig. 3     eine Prinzipskizze eines weiteren Beispiels;

Fig. 4     ein weiteres Ausführungsbeispiel eines erfindungsgemäßen Stechgeräts ohne Gehäuse in einer Seitenansicht;

Fig. 5     das in Figur 4 gezeigte Ausführungsbeispiel in einer Querschnittsansicht;

Fig. 6     ein weiteres Beispiel eines erfindungsgemäßen Stechgeräts in einer Seitenansicht;

Fig. 7     das in Figur 6 dargestellte Beispiel in einer perspektivischen Darstellung;

Fig. 8     das in Figur 6 dargestellte Beispiel beim Spannen der Antriebsfeder;

Fig. 9     das in Figur 6 dargestellte Beispiel in gespanntem Zustand;

Fig. 10    das in Figur 6 dargestellte Beispiel zu Beginn der Vortriebsphase;

Fig. 11    das in Figur 6 dargestellte Beispiel am Ende der Vortriebsphase;

Fig. 12    das in Figur 6 dargestellte Beispiel während der Sammelphase; und

Fig. 13    das in Figur 6 dargestellte Beispiel bei der abschließenden Rückzugsphase nach dem Ende der Sammelphase.

Fig. 14    ein weiteres Beispiel eines erfindungsgemäßen Stechgeräts in einer Seitenansicht;

Fig. 15    das in Figur 14 dargestellte Beispiel in einer Explosionsdarstellung;

Fig. 16    die Steuereinrichtung des in Figur 14 dargestellten Beispiels zu Beginn der Vortriebsphase;

Fig. 17    die in Figur 16 dargestellte Steuereinrichtung am Ende der Vortriebsphase;

Fig. 18    die in Figur 16 dargestellte Steuereinrichtung am Ende der Rückzugsphase;

Fig. 19    die in Figur 16 dargestellte Steuereinrichtung während der Sammelphase;

Fig. 20    die in Figur 16 dargestellte Steuereinrichtung während der weiteren Rückzugsphase;

Fig. 21    eine Darstellung gemäß Figur 16 beim Spannen der Antriebsfeder;

Fig. 22    eine Querschnittsansicht des in Figur 14 dargestellten Ausführungsbeispiels bei maximaler Einstellung der Einstechtiefe;

Fig. 23    eine entsprechende Querschnittsdarstellung bei minimal eingestellter Einstechtiefe;

Fig. 24    ein weiteres Ausführungsbeispiel in einer Querschnittsdarstellung;

Fig. 25    ein weiteres Ausführungsbeispiel in einer Schrägansicht;

Fig. 26    das in Figur 25 dargestellte Ausführungsbeispiel in einer Querschnittsansicht;

Fig. 27    das in Figur 25 dargestellte Ausführungsbeispiel in einer Seitenansicht;

Fig. 28    ein weiteres Ausführungsbeispiel eines Antriebs mit einem Dämpfungsmechanismus;

Fig. 29    ein weiteres Ausführungsbeispiel eines erfindungsgemäßen Stechsystems in einer Seitenansicht;

Fig. 30   das in Figur 29 dargestellte Ausführungsbeispiel in einer Querschnittsansicht; und

Fig. 31   das in Figur 29 dargestellte Ausführungsbeispiel in einer Schrägansicht.

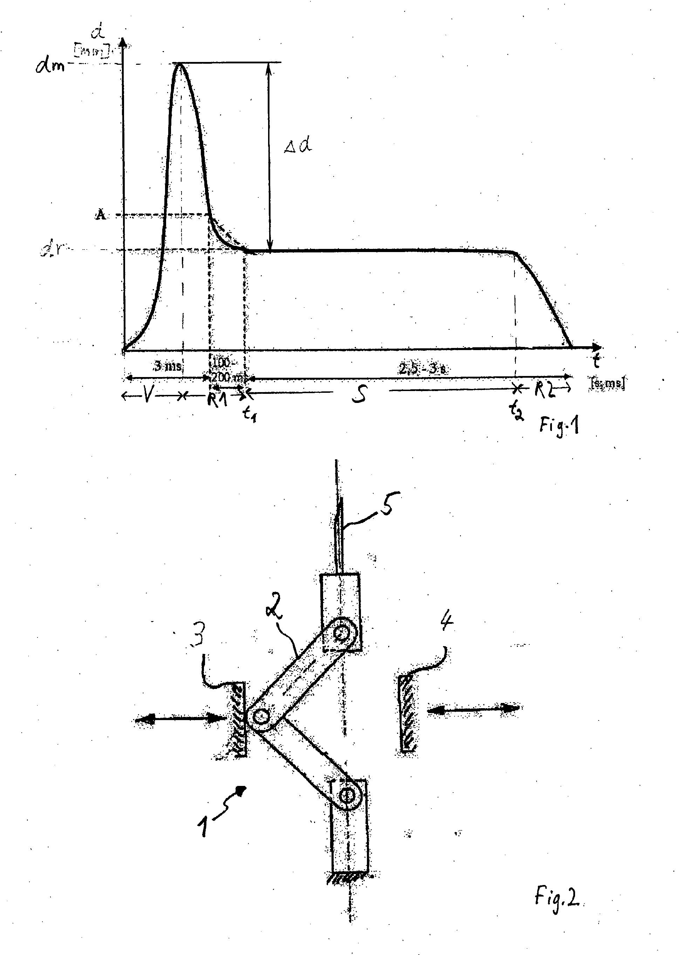

In Figur 1 ist der zeitliche Verlauf der Einstechtiefe d eines Stechelements während der Vortriebsphase V, der Rückzugsphase R1 und der Sammelphase S dargestellt. Die Null-Linie entspricht der Position der Hautoberfläche. Da die Vortriebs- und die Rückzugsphase innerhalb von wenigen Millisekunden ablaufen, während die Sammelphase mehrere Sekunden andauert, ist die Zeitachse in Figur 1 im Bereich der Vortriebsphase V und der Rückzugsphase R1 gestreckt und im Bereich der Sammelphase S gestaucht dargestellt.

Am Ende der Vortriebsphase V erreicht das Stechelement eine maximale Einstechtiefe dm, die je nach Einstellung typischerweise 0,8 mm bis 2,3 mm beträgt. An die Vortriebsphase V schließt eine Rückzugsphase R1 an, in der das Stechelement um eine Rückzugsstrecke Δd teilweise zurückgezogen und gegen Ende der Rückzugsphase abgebremst wird, so daß es mit einer vorgegebenen Reststechtiefe dr von beispielsweise 0,5 mm in die Haut hineinragt.

Für einen schmerzarmen Einstich ist es wesentlich, daß das Stechelement während der Vortriebsphase V sehr schnell eingestochen und sehr schnell zumindest einen Teil der Rückzugsstrecke Δd wieder zurückgezogen wird. Bevorzugt werden dabei Geschwindigkeiten von mindestens 200 mm/sec, besonders bevorzugt mindestens 500 mm/sec, erreicht. Auf einem weiteren Teil der Rückzugsstrecke Δd wird das Stechelement für die Sammelphase abgebremst, so daß der letzte Rest der Rückzugsstrecke Δd wesentlich langsamer durchlaufen wird. Bei dem in Figur 1 dargestellten Verlauf werden die Vortriebsphase V und der erste Teil der Rückzugsstrecke in etwa 3 ms durchlaufen. Die restliche Rückzugsphase (beginnend ab der Einstechtiefe A) dauert etwa 100 ms bis 200 ms. Am Ende der Rückzugsphase R1 wird das Stechelement abgestoppt, so daß sich für die Sammelphase S eine konstante Reststechtiefe dr ergibt.

Während der Sammelphase wird eine Körperflüssigkeitsprobe, in der Regel Blut oder interstitielle Flüssigkeit, durch einen Kapillarkanal der Probenentnahmeeinheit aufgenommen. Vorzugsweise ist der Kapillarkanal zumindest auf einem Teil seiner Länge seitwärts offen (US 2003/0018282 A1). Die Sammelphase dauert typischerweise etwa 1 bis 3 sec. Im Anschluß an die Sammelphase wird das Stechelement erneut beschleunigt und in einer zweiten Rückzugsphase R2 vollständig aus der Haut herausgezogen.

Ein Beispiel einer Konstruktion, mit der sich das in Figur 1 dargestellte Stechprofil verwirklichen läßt, ist in Figur 2 dargestellt. Die Steuereinrichtung 1 umfaßt ein Schwenkelement 2 in Form eines zwischen zwei seitlichen Anschlägen 3, 4 angeordneten Kniehebels. Um eine Einführ- und Rückführbewegung des Stechelements 5 zu bewirken, geht der Kniehebel 2 unter Einwirkung einer Antriebskraft aus der in Figur 2 dargestellten gebeugten Anfangsposition in eine gestreckte Zwischenposition und anschließend in eine umgekehrt gebeugte Endposition über, bei der er an dem Endanschlag 4 anliegt. In der gestreckten Zwischenposition (nicht dargestellt) erreicht das Stechelement 5 seine maximale Einstechtiefe.

Durch die Position des Endanschlags 4 läßt sich einstellen, wie weit das Stechelement 5 bei der Rückzugsphase zurückgezogen wird. Je weiter der Endanschlag 4 in Figur 2 nach rechts verschoben wird, desto größer ist die Rückzugsstrecke Δd während der Rückzugsphase R1. Der Endanschlag 4 bildet deshalb bei dem dargestellten Ausführungsbeispiel eine Rückführsperre, mit der das Stechelement 5 am Ende der Rückzugsphase R1 abgestoppt wird. Nach dem Ende der Sammelphase kann der Endabschlag 4 in eine Endposition (d.h. bei der in Figur 1 dargestellten Anordnung ganz nach rechts) bewegt werden, so daß das Stechelement 5 in der Rückzugsphase R2 vollständig aus der Haut herausgezogen wird.

Die Antriebskraft zum Bewegen des Kniehebels 2 wird mit einer Antriebsfeder (nicht dargestellt) erzeugt. Während der Einstichbewegung wird das Kniegelenk mittels der Federkraft von dem Anfangsanschlag 3 zu dem Endanschlag 4 verschoben.

Die Tiefeneinstellung erfolgt in zwei Schritten: Der Anfangsanschlag 3 wird so eingestellt, daß die größte Bewegungsweite des Stechelementes 5 der maximalen Einstechtiefe entspricht. Vorzugsweise gleichzeitig und damit gekoppelt wird der Endanschlag 4 so eingestellt, daß der Rückweg des Stechelementes um die Reststechtiefe geringer ist.

Gemäß einer vorteilhaften Ausgestaltung kann bei dieser Ausführungsform, wie auch generell bei der vorliegenden Erfindung, vorgesehen sein, daß die Position der Hautoberfläche vor dem Auslösen einer Einstechbewegung detektiert und die nachfolgende Einstechbewegung an die detektierte Position angepaßt wird. Dadurch ist es möglich, eine reproduzierbare maximale Stechtiefe auch dann sicherzustellen, wenn sich die Haut beim Andrücken des Stechgerätes aufgrund ihrer Elastizität in unterschiedlichem Ausmaß in die (hier nicht dargestellte) vordere Gehäuseöffnung des Stechgerätes hineinwölbt ("Z-Varianz"). Eine solche Vorabdetektion der Hautoberfläche kann beispielsweise dadurch realisiert werden, daß der in Figur 2 dargestellte Mechanismus auf einer Linearführung nach vorne (in Stichrichtung) gefahren wird, bis die Spitze des Stechelementes 5 die Hautoberfläche berührt, wobei diese Berührung elektronisch detektiert werden kann. Eine solche Konstruktion wird im Zusammenhang mit den Figuren 29 bis 31 erläutert. Der gleiche Antrieb, der für die Vorabdetektion verwendet wird, kann auch zum Herausziehen des Nadelelementes in der zweiten Rückzugsphase R2 verwendet werden.

Um die Position des Anfangsanschlages 3 und des Endanschlags 4 simultan einzustellen, kann beispielsweise ein Plangewinde verwendet werden, wie es in Backenfuttern von Drehmaschinen üblich ist. Bei der Einstellung ist zu beachten, daß ein nichtlinearer Zusammenhang zwischen dem Verschiebeweg der Anschläge 3,4 und der Stechtiefe besteht. Um das Einstellen zu erleichtern, kann diese Nichtlinearität beispielsweise durch eine geeignete Nockenform oder Steuerkulisse ausgeglichen werden. Die Position der Anschläge 3,4 kann manuell oder prozessorgesteuert mit einem geeigneten Elektromotor, der beispielsweise auch zum Spannen der Antriebsfeder genutzt werden kann, eingestellt werden.

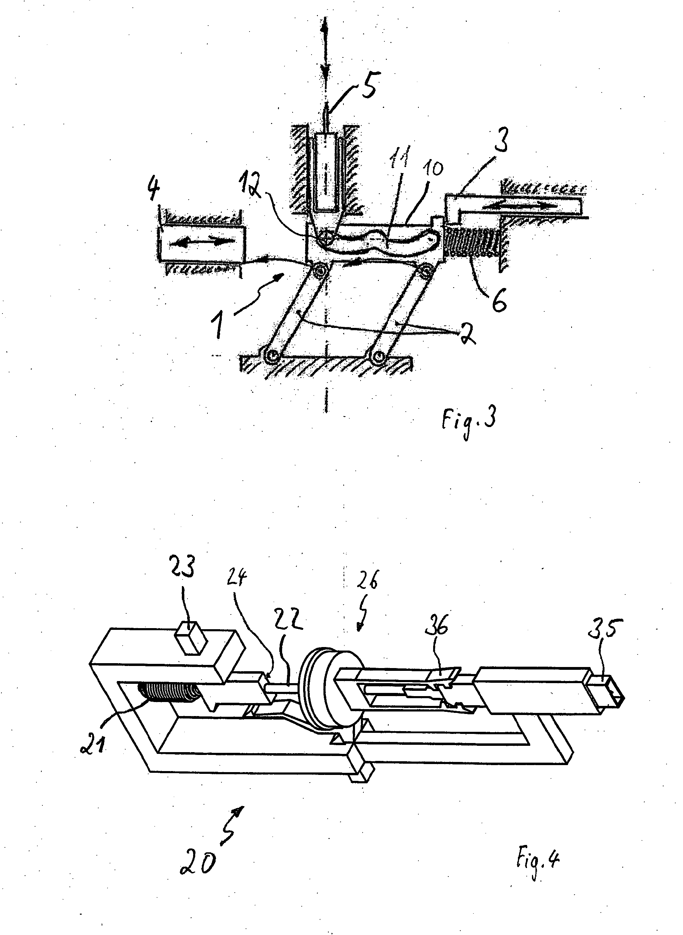

In Figur 3 ist ein weiteres Beispiel einer Konstruktion dargestellt, mit der sich das in Figur 1 dargestellte Stechprofil verwirklichen läßt. Die Steuereinrichtung 1 umfaßt ein Schwenkelement 2 in Form einer zwischen zwei seitlichen Anschlägen 3, 4 angeordneten Parallelogrammführung. Um eine Einführ- und Rückführbewegung des Stechelements 5 zu bewirken, geht die Parallelogrammführung 2 unter Einwirkung der von der Antriebsfeder 6 erzeugten Antriebskraft aus der in Figur 3 dargestellten Anfangsposition in eine Endposition über, in der sie an dem Endanschlag 4 anliegt.

Die Parallelogrammführung 2 umfaßt ein Steuerelement 10 mit einer Steuerkurve 11, die bei einer Einstich- und Rückführbewegung von einem Steuerkurvenreiter 12 abgefahren wird, so daß die Schwenkbewegung der Parallelogrammführung 2 in eine lineare Einstich- und Rückführbewegung des Stechelements 5 umgesetzt wird. Bei dem dargestellten Ausführungsbeispiel ist die Steuerkurve 11 als Nut ausgebildet, in die ein als Zapfen ausgebildeter Steuerkurvenreiter 12 eingreift.

Wie bei dem anhand von Figur 2 beschriebenen Ausführungsbeispiel läßt sich die Position der Anschläge 3 und 4 einstellen, um zu definieren, wie weit das Stechelement 5 vor- und zurückbewegt wird. Durch die Form der Steuerkurve 11 kann dabei erreicht werden, daß zwischen der Position der Endanschläge 3 und 4 und dem Stichhub ein linearer Zusammenhang erzielt wird, der das Einstellen der Einstechtiefe erleichtert.

Bei dem dargestellten Ausführungsbeispiel wird während der Vortriebsphase V und der Rückzugsphase R1 nicht die gesamte Steuerkurve 11, sondern lediglich ein aktiver Abschnitt der Steuerkurve 11 von dem Steuerkurvenreiter 12 abgefahren. Anfang und Ende des aktiven Abschnitts werden dabei durch die Positionen der Anschläge 3 und 4 vorgegeben. Die Funktion entspricht im wesentlichen der in Figur 2 dargestellten Ausführungsform.

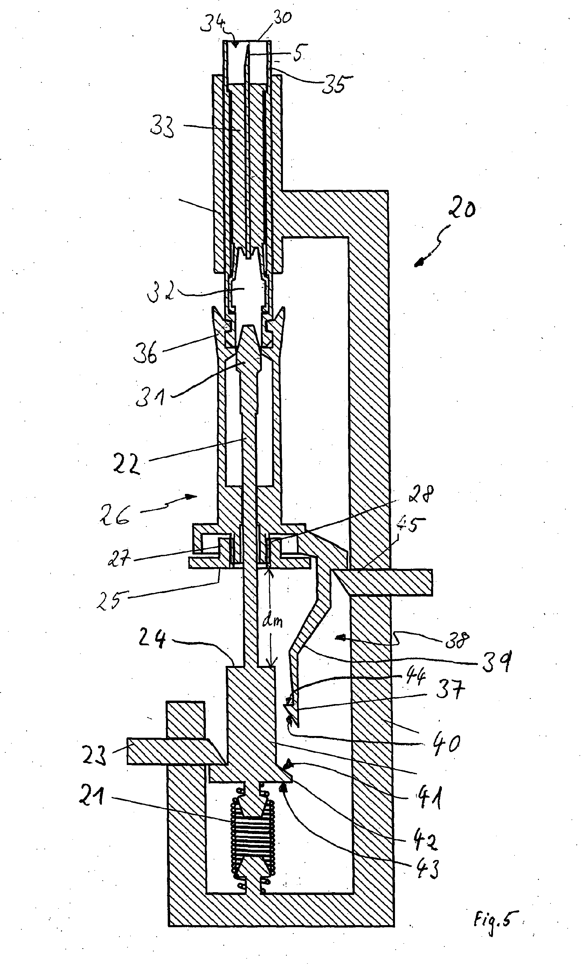

In den Figuren 4 und 5 ist ein weiteres Beispiel eines Stechgeräts 20 dargestellt, mit dem sich das in Figur 1 dargestellte Stechprofil verwirklichen läßt. Zur Vereinfachung sind nur die wesentlichen Teile des Stechgeräts 20 ohne vollständiges Gerätegehäuse dargestellt.

Das Stechgerät 20 hat einen Federantrieb mit einer Antriebsfeder 21. Die Antriebsfeder 21 ist eine Schraubenfeder, die in den Figuren 4 und 5 in komprimiertem Zustand dargestellt ist. Die Antriebsfeder 21 ist mit einer Schubstange 22 gekoppelt, die von der Antriebsfeder 21 in Einstichrichtung beschleunigt wird, sobald ein Triggerelement 23 betätigt wird: Bei dem Triggerelement 23 handelt es sich um einen Riegel, der in der dargestellten Halteposition die Schubstange 22 arretiert. Zum Auslösen wird der Riegel so zurückgezogen, daß die Schubstange 22 von der Antriebsfeder 21 in Einstichrichtung beschleunigt werden kann.

Während der Vortriebsphase V bewegt sich die Schubstange 22, bis sie am Ende der Vortriebsphase V mit einem an der Schubstange 22 vorgesehenen Anschlag 24 gegen einen Anschlag 25 einer Einstelleinrichtung 26 prallt. Der Anschlag 25 ist als Kopf einer Einstellschraube 27 ausgebildet, deren Gewinde 28 parallel zu der Einstichrichtung verläuft. Durch Drehen der Einstellschraube 27 kann die Längsposition des Anschlags 25 relativ zu einer an der Haut anliegenden Referenzfläche 30 eingestellt werden. Durch die Längsposition des Anschlags 25 und die entsprechende Längsposition der Referenzfläche 30 in Relation zu der Längsposition der Spitze des Stechelementes 5 ist die Einstechtiefe einer bei einem Einstich erzeugten Einstichwunde vorgegeben. "Längsposition" bezeichnet dabei jeweils die Position in der Richtung, in der der Einstich durchgeführt wird.

Die Schubstange 22 hat einen Kopf 31, der während der Einstichbewegung in eine passende Kupplungsausnehmung 32 eines Lanzettenkörpers 33 eintaucht und dabei mit der Lanzette 34 fest gekoppelt wird. Die in den Figuren 4 und 5 dargestellte Lanzette 34 umfaßt ein Stechelement 5, das in einem Lanzettenkörper 33 aus Kunststoff eingebettet ist. Das Stechelement 5 hat einen Kapillarkanal, so daß mit der Lanzette 34 als Probenentnahmeeinheit während einer Sammelphase S Körperflüssigkeit aufgenommen werden kann. Die Lanzette 34 wird zusammen mit einem hülsenförmigen Stechtiefenreferenzelement 35 in das Stechgerät 20 eingesetzt. Das Stechtiefenreferenzelement 35 liegt mit seinem vorderen Ende bei einem Einstich an der Hautoberfläche an und bildet auf diese Weise einen präzisen Bezugspunkt zur Definition der Einstechtiefe.

Zu diesem Zweck ist eine exakte Längspositionierung des Referenzelements 35, relativ zu der Einstelleinrichtung 26 notwendig. Sie wird im dargestellten Fall dadurch erreicht, daß das Stechtiefenreferenzelement 35 bei korrekter Positionierung mit Rastelementen 36 der Einstelleinrichtung 26 verrastet. Bei dem dargestellten Ausführungsbeispiel hat das Referenzelement 35 zu diesem Zweck an seinem hinteren Ende eine Nut, in die als Rasthaken ausgebildete Rastelemente 36 eingreifen.

Wenn die Schubstange 22 am Ende der Vortriebsphase V mit ihrem Anschlag 24 den Anschlag 25 der Einstelleinrichtung 26 kontaktiert, ist die Antriebsfeder 21 gestreckt, so daß die Schubstange 22 während der Rückzugsphase R1 von der Antriebsfeder 21 wieder zurückgezogen wird. Die Federkraft der Antriebsfeder 21 dient also sowohl zum Beschleunigen der Schubstange 22 während der Vortriebsphase V als auch zum Beschleunigen der Schubstange 22 in umgekehrte Richtung während der Rückzugsphase R1.

Am Ende der Rückzugsphase R1 wird die Schubstange 22 und damit auch die mit ihr verrastete Lanzette 34 mit dem Stechelement 5 durch ein Sperrelement 37 einer Rückführsperre 38 abgestoppt. Dabei wird die Reststechtiefe dr (bei gegebener Position des Referenzelements 35) durch die Längsposition der Rückführsperre 38 in Einstichrichtung definiert. Als Rückführsperre eignet sich grundsätzlich jedes Element, durch das die Rückführbewegung des Stechelementes 5 in einer definierten Längsposition gestoppt werden kann.

Bei der dargestellten Ausführungsform umfaßt die Rückführsperre 38 ein Federelement 39 in Form eines Federarms, der an seinem freien Ende das Sperrelement 37 in Form einer Sperrklinke trägt. Die Sperrklinke 37 hat eine abgeschrägte Gleitfläche 40, an der während der Vortriebsphase V eine Gleitfläche 41 eines an der Schubstange 22 angeordneten Rastvorsprungs 42 vorbeigleitet. Bei diesem Vorbeigleiten während der Vortriebsphase V gibt der Federarm 39 der Rückführsperre 37 nach.

Sobald der Rastvorsprung 42 das Sperrelement 37 passiert hat, kehrt dieses wegen der von dem Federarm 37 aufgebrachten Federkraft wieder in seine ursprüngliche Position zurück, in der es am Ende der Rückzugsphase die Schubstange 22 abstoppt, wobei ein Anschlag 43 des Rastvorsprungs 42 der Schubstange 22 gegen einen korrespondierenden Anschlag 44 des Sperrelements 37 stößt.

Bei dem dargestellten Ausführungsbeispiel ist die Rückführsperre 38 gegenüber dem Gerätegehäuse (nicht dargestellt) beweglich und wird während der Sammelphase S durch einen Sperriegel 45 fixiert. Am Ende der Sammelphase wird der Sperriegel 45 aus der in Figur 5 dargestellten Eingriffsposition in eine Rückzugsposition zurückgezogen, so daß die Rückführsperre 38 zusammen mit der Schubstange 22 von der Antriebsfeder 21 zurückgezogen wird. Durch Zurückziehen des Sperriegels 45 wird also die zweite Rückzugsphase R2 eingeleitet.

Wie bereits erwähnt, verrastet der Kopf der Schubstange 31 formschlüssig mit der Lanzette 34, so daß über die Schubstange 22 nicht nur Schub-, sondern auch Zugkräfte auf die Lanzette 34 übertragen werden können. Bei dem dargestellten Ausführungsbeispiel ist die Rückführsperre 38 fest mit den Rastelementen 36 der Einstelleinrichtung 26 verbunden, mit denen die Lanzettenhülse 35 verrastet ist. Besonders günstig ist eine einstückige Ausführung der Rückführsperre 38 mit den Rastelementen 36 als Spritzgußteil. Auf diese Weise wird während der weiteren Rückzugsphase R2 zusammen mit der Rückführsperre 38 und der Schubstange 22 auch das Stechtiefenreferenzelement 35 zurückgezogen.

Durch das Anpressen des Referenzelements 35 kann der Austritt von Körperflüssigkeit aus einer Einstichwunde behindert werden. Andererseits kann ein starker Anpressdruck bei dem Einstich zu einem verminderten Schmerzempfinden beitragen. Eine wichtige Besonderheit des beschriebenen Ausführungsbeispiels besteht deshalb darin, daß das Referenzelement 35 am Ende der Vortriebsphase zusammen mit der Lanzette vorgeschoben wird. Wird das Referenzelement 35 vor Auslösen des Einstichs an die Hautoberfläche des Benutzers angelegt oder während der Vortriebsphase in Richtung auf die Hautoberfläche bewegt, wird auf diese Weise der Anpressdruck während des Einstichs erhöht. Die Einstechtiefe wird dabei durch den Abstand definiert, um den am Ende der Vortriebsphase das Stechelement 5 über die Referenzfläche 30 hinausragt, mit der das Referenzelement 35 an der Hautoberfläche anliegt. Während der Rückzugsphase werden das Referenzelement 35 und das Stechelement 5 zurückgezogen, so daß der Anpressdruck vermindert wird und das Stechelement mit einer vorgegebenen Reststechtiefe in die Haut hineinragt. Wegen der elastischen Eigenschaften der Hautoberfläche kann die Hautoberfläche auch nach dem Teilrückzug an dem Referenzelement 35 anliegen.

Die in den Figuren 4 und 5 dargestellte Ausführungsform zeigt somit beispielhaft, daß es vorteilhaft sein kann, wenn das Stechtiefenreferenzelement 35 nur in dem dem Umkehrpunkt unmittelbar vorausgehenden Teil der Vortriebsphase gemeinsam (d.h. gleichzeitig, nicht notwendigerweise gleich schnell) mit dem Stechelement 5 bewegt wird. Vorzugsweise beträgt der Hub (Wegstrecke in Einstechrichtung), um den das Stechtiefenreferenzelement 35 bis zum Erreichen des Umkehrpunktes gemeinsam mit dem Stechelement 5 bewegt wird, höchstens 5 mm, vorzugsweise höchstens 3,5 mm und besonders bevorzugt höchstens 2 mm. Bei der dargestellten Konstruktion wird dies dadurch realisiert, daß das Stechtiefenreferenzelement 35 während des größten Teils der Stechbewegung des Stechelements 5 in einer Ruheposition auf einem Referenzelementlager ruht und ausgehend von dieser Ruheposition in Richtung auf die Haut beschleunigt wird, kurz bevor das Stechelement 5 den Umkehrpunkt zwischen Einstich- und Rückführbewegung erreicht. Eine andere Konstruktion, die ein ähnliches Bewegungsverhalten bewirkt, ist in den Figuren 29 bis 31 dargestellt.

Weiterhin ist es vorteilhaft, wenn der Teilabschnitt der Stechbewegung, während dessen das Stechtiefenreferenzelement 35 gemeinsam mit dem Stechelement 5 während der Vortriebsphase (also bis zum Erreichen des Umkehrpunktes) bewegt wird, in sehr kurzer Zeit zurückgelegt wird. Vorzugsweise sollte diese Zeitdauer maximal 100 msec, bevorzugt maximal 50 msec und besonders bevorzugt maximal 10 msec betragen.

Im Rahmen der Erfindung wurde festgestellt, daß durch die vorstehend genannten Maßnahmen, die einzeln oder in Kombination eingesetzt werden können, einerseits die Stechtiefe präzise kontrolliert werden kann, andererseits die viskoelastischen Eigenschaften der Haut bei einem derartig kurzen Anpressen nicht dazu führen, daß die präzise Definition der Reststechtiefe durch eine Verformung der Haut beeinträchtigt wird. Ergänzend ist es vorteilhaft, wenn bei einem Stechprofil der in Figur 1 dargestellten Art die erste Rückzugsphase R1 zwischen dem Erreichen des Umkehrpunktes (maximale Stechtiefe) und dem Beginn der Sammelphase maximal 2 sec, bevorzugt maximal 1 sec und besonders bevorzugt maximal 0,5 sec dauert.

Bei dem in den Figuren 4 und 5 dargestellten Ausführungsbeispiel ist die Reststechtiefe unabhängig von der eingestellten Einstechtiefe bei jeder Probenentnahme konstant.

Als günstig haben sich Reststechtiefen von 0,3 mm bis 0,6 mm, bevorzugt 0,4 mm bis 0,5 mm erwiesen. Damit bei dem dargestellten Stechgerät 20 eine definierte Reststechtiefe erreicht wird, ist es wichtig, daß durch die Rückführsperre 38 die Position des Stechelements 5 relativ zu dem an der Haut anliegenden Referenzelement 35 definiert wird. Bei dem dargestellten Ausführungsbeispiel wird dies dadurch erreicht, daß die Lanzette 34 durch die Rückführsperre 38 gegenüber dem Referenzelement 35 arretiert wird. Für die Rückführbewegung kann gemäß dem dargestellten Ausführungsbeispiel ebenfalls die Antriebsfeder 21 verwendet werden. Möglich ist es aber auch, für die Rückführbewegung eine weitere Feder vorzusehen. Für den vollständigen Rückzug des Stechelements 5 aus der Haut kann beispielsweise ein Linearantrieb verwendet werden, der auch zum Spannen der Antriebsfeder 21 vor einem Einstich und zur Vorabdetektion der Z-Position der Hautoberfläche (wie im Hinblick auf Figur 2 erläutert) verwendet werden kann. Alternativ kann die Kraft für das vollständige Zurückziehen des Stechelements 5 aus der Haut auch an der Rückführsperre 38 selbst oder der Lanzettenhülse 35 ansetzen.

Die in den Figuren 4 und 5 dargestellte Konstruktion ist ein Beispiel für eine Ausführungsform der Erfindung, bei der die Antriebsfeder 21 direkt nur mit dem Stechelement 5 gekoppelt ist, während das Referenzelement 35 über eine in der Vortriebsphase der Stechbewegung wirkende Mitnehmereinrichtung mit dem Stechelement 5 und dadurch indirekt mit der Antriebsfeder 21 gekoppelt ist. Bestandteile der Mitnehmereinrichtung sind die beiden Anschläge 24 und 25, die während der weiteren Vortriebsphase bis zum Erreichen des Umkehrpunktes zwischen Einstich- und Rückführbewegung derartig aneinander anliegen, daß ihr relativer Abstand die Längsposition des Stechelementes 5 relativ zu dem Referenzelement 35 und damit die Stechtiefe definiert. Die Anschläge 24,25 werden deshalb als Stechtiefenbegrenzungsanschläge bezeichnet.

Im dargestellten Fall wirkt die Mitnehmereinrichtung bidirektional, wobei sie je einen mit dem Stechelement 5 bzw. dem Referenzelement 35 gekoppelten in der Rückzugsphase wirkenden Anschlag 43,44 aufweist und die Anschläge während mindestens eines Teils der Rückzugsphase derartig aneinander anliegen, daß

dadurch die Position des Stechelementes 5 relativ zu dem Referenzelement 10 definiert ist.

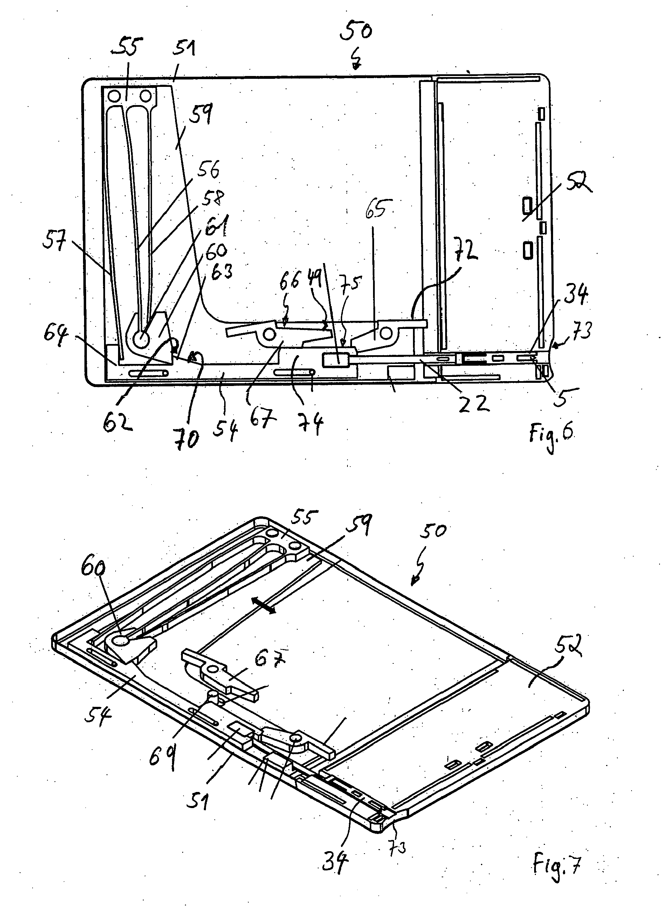

Ein weiteres Ausführungsbeispiel eines Stechgeräts 50, mit dem sich das in Figur 1 dargestellte Stechprofil verwirklichen läßt, ist in den Figuren 6 und 7 in einer Seitenansicht jeweils bei geöffnetem Gehäuse 51 dargestellt. Das dargestellte Stechgerät 50 zeichnet sich durch seine flache Bauweise mit einer Bauhöhe von weniger als 4 mm aus. Zu dem dargestellten Stechgerät 50 gehört ein Lanzettenmagazin 52, in dem Lanzetten 34 nebeneinander gelagert sind und durch eine Magazinfeder (nicht dargestellt) zum Gebrauch nacheinander in eine Gebrauchsposition, in der sie an eine Schubstange 22 ankoppeln können, aus dem Magazin 52 herausgeschoben werden. Die Lanzetten 34 umfassen in der üblichen Weise einen Kunststoffkörper, in dem ein Stechelement 5 eingebettet ist, das zur Probenaufnahme einen oder mehrere Kapillarkanäle aufweisen kann. Die Schubstange ist an einem Schlitten 54 befestigt, der mittels eines Federantriebs 55 für eine Einstich- und Rückführbewegung beweglich ist.

Zu dem Federantrieb 55 gehört eine erste Blattfeder 56 als Antriebsfeder, eine zweite Blattfeder 57 als Rückzugsfeder und eine dritte Blattfeder 58 als Klinkenfeder.

Die Blattfedern 56, 57, 58 sind an einem Ende an einer gegenüber dem Gerätegehäuse 51 beweglichen Basisplatte 59 befestigt. Die Antriebsfeder 56 trägt an ihrem freien Ende eine drehbare Klinke 60, über die eine Bewegung der Antriebsfeder 56 auf den Schlitten 55 übertragen wird. Die Antriebsfeder 56 hat zu diesem Zweck an ihrem freien Ende einen zylindrischen Kopf 61, der in einer passenden Ausnehmung der Klinke 60 sitzt. Die Klinke 60 tritt mit dem Schlitten 54 über eine Ansatzfläche 62 in Wechselwirkung, die bei dem dargestellten Ausführungsbeispiel an einem Vorsprung 63 des Schlittens 54 angeordnet ist. In Figur 6 ist das Stechgerät in seinem gespannten Zustand vor dem Auslösen eines Einstichs dargestellt.

Die Klinkenfeder 58 liegt mit ihrem freien Ende an der Klinke 60 an und übt dabei auf die Klinke 60 ein Drehmoment aus, das bei dem dargestellten Ausführungsbeispiel im Uhrzeigersinn gerichtet ist. Dieses Drehmoment sorgt dafür, daß die Klinke 60 zuverlässig mit dem Schlitten 54 in Wirkeingriff steht.

Die Rückholfeder 57 wirkt der Antriebsfeder 56 entgegen, übt also auf den Schlitten 54 eine in Rückführrichtung gerichtete Kraft aus. Bevorzugt liegt die Rückholfeder 57 an einem Rückführelement 64 des Schlittens 54 an, das bei dem dargestellten Ausführungsbeispiel als Vorsprung ausgebildet ist.

Die Rückholfeder 57 kann durch eine Schraubenfeder ersetzt werden, die an dem Gerätegehäuse 51 und dem Schlitten 54 befestigt ist. Der dargestellte Antrieb 55, bei dem die Antriebsfeder 56, die Klinkenfeder 58 und die Rückholfeder 57 einstückig als Stanzteil aus Federstahl hergestellt sind, hat jedoch den Vorteil, besonders kostengünstig zu sein und sich leicht montieren zu lassen.

Zur Einstellung der Einstechtiefe wird die Basisplatte 59 mittels einer Einstelleinrichtung (nicht dargestellt) gegenüber dem Gehäuse 51 verschoben. Außer den bereits beschriebenen Bauteilen trägt die Basisplatte 59 eine Sperrklinke 65, die in der in Figur 6 dargestellten gespannten Position den Schlitten 54 arretiert.

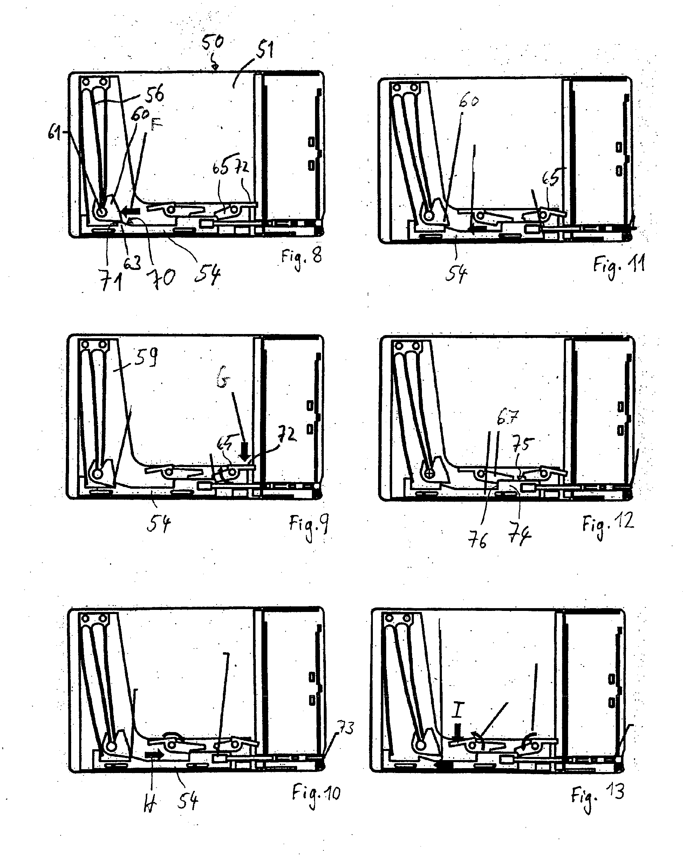

Zum Abstoppen des Stechelements 5 am Ende der Rückzugsphase R1 dient eine Rückführsperre 66 mit einem Sperrelement in Form einer weiteren Sperrklinke 67, deren Funktion anhand der Figuren 8 bis 13, die einen vollständigen Arbeitszyklus des Stechgeräts darstellen, erläutert wird.

Damit die Einstechtiefe unabhängig von der Reststechtiefe eingestellt werden kann, ist es wichtig, daß die Sperrklinke 67 der Rückführsperre 66 nicht an der Basisplatte 59 befestigt ist, die zur Einstellung der Einstechtiefe in dem Gerätegehäuse 51 verschoben wird, sondern relativ zu dem Gehäuse fest, oder gesondert einstellbar, gelagert ist. In Figur 7 ist die Sperrklinke 67 abgehoben von einem Zapfen 69 dargestellt.

In Figur 8 ist das Stechgerät 50 mit entspannter Antriebsfeder 56 dargestellt. Zum Spannen wird zunächst die Klinke 60 in Pfeilrichtung F zurückgeschoben. Der dafür erforderliche Schiebemechanismus ist der besseren Übersichtlichkeit wegen nicht dargestellt. Geeignet ist beispielsweise ein einfacher Schieber, der mit einem Betätigungselement durch einen Schlitz aus dem Gerätegehäuse 51 herausragt und von einem Benutzer mit dem Finger in Pfeilrichtung F verschoben werden kann.

Beim Zurückschieben gleitet die Klinke 60 an einer schrägen Gleitfläche 70 des Schlittenvorsprungs 63 entlang, bis die durch einen Hinterschnitt gebildete Schubfläche 71 erreicht wird. Durch die Klinkenfeder 58 wird die Klinke 60 dann in die in Figur 7 dargestellte Position gebracht, in der sie an der Schubfläche 71 anliegt und auf diese Weise mit dem Schlitten 54 in Wirkeingriff steht. Das Stechgerät 50 ist nun gespannt. Die von der Antriebsfeder über die Klinke auf den Schlitten ausgeübte Kraft wird in diesem Zustand von der Sperrklinke 65 aufgenommen, so daß der Schlitten 54 bis zum Auslösen eines Einstichs in der in Figur 7 dargestellten Position arretiert ist.

Zum Auslösen wird auf die Sperrklinke 65 in Richtung des Pfeils G eine Kraft ausgeübt, so daß die drehbar an der Basisplatte 59 befestigte Sperrklinke 65 aus ihrer in Figur 7 dargestellten Sperrposition gedreht wird. Der Auslösemechanismus zum Betätigen der Sperrklinke 65 ist der besseren Übersichtlichkeit halber nicht dargestellt. Beispielsweise kann es sich dabei um einen Stift handeln, der durch eine Öffnung in der Gehäuseseite aus dem Gehäuse herausragt, so daß er von einem Benutzer gedrückt werden kann. Alternativ kann der Betätigungsmechanismus auch einen Aktuator, z.B. aus einer Formgedächtnislegierung, umfassen. Dadurch wird die zeitgenaue Steuerung der Sammelphase erleichtert. Die Sperrklinke 65 hat einen langgestreckten Betätigungsarm 72, am dem ein Betätigungselement (nicht dargestellt) ansetzen kann, so daß unabhängig von der Position der Basisplatte 59 durch Drücken eines Betätigungsstifts die Sperrklinke 65 betätigt werden kann.

Sobald die Sperrklinke 65 durch eine Drehung den Schlitten 54 freigibt, wird dieser von der Antriebsfeder 56 in Richtung des Pfeils H beschleunigt, so daß die Schubstange 22 an eine Lanzette 34 ankoppelt und mit dem Stechelement 5 der Lanzette 34 eine Einstichwunde in einem an der Andruckfläche 73 des Gerätegehäuses 51 anliegenden Körperteil, in der Regel einem Finger, erzeugt werden kann. Die Andruckfläche 73 ist als eine muldenförmige Vertiefung in einer Seitenfläche des Gehäuses 51 angeordnet, um einem Benutzer das korrekte Positionieren des betreffenden Körperteils zu erleichtern.

Beim Beschleunigen des Schlittens 54 schwingt die Klinke 60 von der Antriebsfeder 56 getrieben auf eine bogenförmige Bahn in Richtung des Pfeils H. Da die Antriebsfeder 56 an ihrem von der Klinke 60 abgewandten Ende an der Basisplatte 59 befestigt ist, entfernt sich die Klinke 60 dabei zunehmend von dem Schlitten 54, bis sie von dem Schlitten 54 abhebt. Dies ist in Figur 11 dargestellt. Figur 11 zeigt dabei zugleich den Umkehrpunkt der Lanzettenbewegung, d.h. die Position am Ende der Vortriebsphase V.

Während der Vortriebsphase V führt die Sperrklinke 67 der Rückführsperre 66 eine Drehbewegung aus, so daß der Schlitten 54 bei der von der Rückführfeder 57 bewirkten Rückführbewegung in der in Figur 12 dargestellten Position am Ende der Rückführphase R1 gestoppt wird. Diese Drehbewegung der Sperrklinke 67 wird durch das Federelement 49 (Figur 6) bewirkt, das sich der Sperrklinke 67 und dem Gehäuse 51 abstützt. Der Schlitten 54 hat zu diesem Zweck einen Kopf 74 mit einer Gleitfläche 75, an der die Sperrklinke 65 zu Beginn der Vortriebsphase V (Figur 9) anliegt. Der Kopf 74 ist durch eine Stufe 76 begrenzt, an der die Sperrklinke 67 in der in Figur 12 dargestellten Eingriffsposition ansetzt.

Die Sperrklinke 67 der Rückführsperre 66 bleibt während der gesamten Sammelphase S in der in Figur 12 dargestellten Eingriffsposition. Am Ende der Sammelphase S wird die Sperrklinke 67 der Rückführsperre 66 betätigt, so daß der Schlitten 54 freigegeben wird. Der Schlitten 54 wird dann durch eine von der Rückholfeder 57 aufgebrachten Rückführkraft während der weiteren Rückführphase R2 in seine Ausgangsposition zurückgezogen und das Stechelement 5 vollständig aus der Haut herausgezogen.

Der Mechanismus zum Betätigen der Sperrklinke 67 der Rückführsperre 66 ist der besseren Übersichtlichkeit halber nicht dargestellt. In einfachstem Fall kann die Sperrklinke 67 durch eine in Richtung des Pfeils I wirkende Kraft in Drehung versetzt werden. Eine entsprechende Mechanik kann beispielsweise von einem Aktuator aus einer Formgedächtnislegierung gebildet werden. Beispielsweise kann ein derartiger Aktuator durch einen kurzen Strompuls über die Umwandlungstemperatur der Formgedächtnislegierung hinaus erwärmt werden, so daß durch eine dabei bewirkte Formänderung des Aktuators eine in Richtung des Pfeils wirkende Kraft erzeugt werden kann.

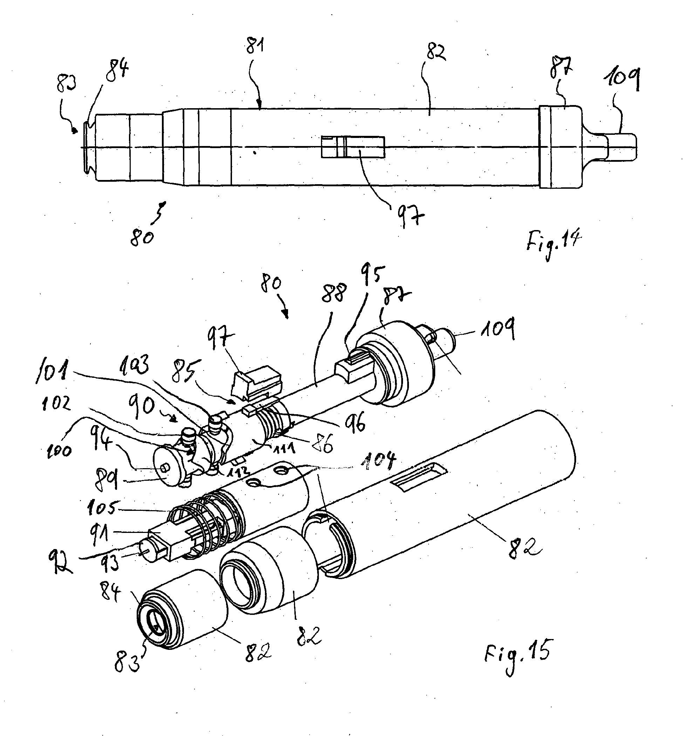

Ein weiteres Ausführungsbeispiel eines Stechsystems 80 ist in Figur 14 in einer Seitenansicht und in Figur 15 in einer Explosionsdarstellung gezeigt. Das zu dem Stechsystem gehörende Stechgerät 81 hat ein Gehäuse 82 mit einer Gehäuseöffnung 83, die von einem Andruckring 84 umgeben ist, der sich beim Andrücken eines Körperteils elastisch deformiert. Beispielsweise kann der Andruckring 84 aus einem gummielastischem Kunststoff hergestellt sein. Bevorzugt hat der Andruckring 84 eine nach innen geneigte Andruckfläche, an die bestimmungsgemäß ein Finger oder ein anderes Körperteil angelegt wird. Ein geeigneter Andruckring 84 ist detailliert in der WO 01/89383 A2 als Kompressionseinheit beschrieben, die diesbezüglich durch Bezugnahme zum Gegenstand der Anmeldung gemacht wird.

Das Stechgerät 81 hat einen Federantrieb 85 mit einer Antriebsfeder 86, die durch Drehen eines aus dem Gehäuse herausragenden Spannknopfes 87 gespannt wird. Der Spannknopf 87 bildet den Kopf eines Spannrotors 88, der mit der Antriebsfeder 86 gekoppelt ist. Die Antriebsfeder 86 ist mit einem Antriebsrotor 89 gekoppelt, dessen Drehbewegung mittels einer Steuereinrichtung 90 in eine Einstich- und Rückführbewegung eines Lanzettenträgers 91 umgesetzt wird. Der Lanzettenträger 91 trägt eine auswechselbare Probenentnahmeeinheit 92, die als Lanzette mit einem in einem Kunststoffkörper eingebetteten Stechelement 93 ausgebildet ist.

Nach Gebrauch kann eine Probenentnahmeeinheit 91 mittels einer Auswurfstange 94 von dem Lanzettenträger gelöst und aus der Gehäuseöffnung 83 heraus geschoben werden. Die Auswurfstange 94 ist durch eine zentrale Öffnung der von dem Spannrotor 88 und dem Antriebsrotor gebildeten Antriebsbaugruppe hindurchgeführt, so daß durch drücken eines Auswurfknopfes, der als ein aus dem Gehäuse 82 heraus ragender Kopf 109 der Auswurfstange 94 gebildet ist, eine gebrauchte Probenentnahmeeinheit 92 ausgeworfen werden kann.

Der Spannrotor 89 trägt eine Sperrklinke 95, die mit einer an der Innenwand des Gehäuses 82 ausgebildeten Ratsche zusammenwirkt, so daß sich der Spannrotor 88 nur in einer Richtung drehen kann. Beim Spannen der Antriebsfeder 86 wird eine Bewegung des Antriebsrotors 89 durch eine Sperreinrichtung 96 verhindert. Durch Betätigen des aus dem Gehäuse 82 herausragendem Auslöseelements 97, das bei dem dargestellten Beispiel als Schieber ausgebildet ist, wird die Sperreinrichtung 85 gelöst, so daß sich der Antriebsrotor 89 dreht und eine Einstich- und Rückführbewegung ausgelöst wird.

Auch bei dieser Ausführungsform kann es vorteilhaft sein, die Stechbewegung mittels einer Vorabdetektion der Hautoberfläche an (im Rahmen der "Z-Varianz") unterschiedliche Positionen der Hautoberfläche anzupassen. Dies kann beispielsweise dadurch geschehen, daß nur der Andruckring 84 fest an dem Gehäuse 82 befestigt ist, während die dargestellte Mechanik in dem Gehäuse 82 mit einem Linearantrieb in Längsrichtung bewegt werden kann. Dieser Linearantrieb dient dazu, die Mechanik so einzustellen, daß nach dem Einstich die gewünschte Reststechtiefe gewährleistet ist (vgl. auch Figuren 29 bis 31).

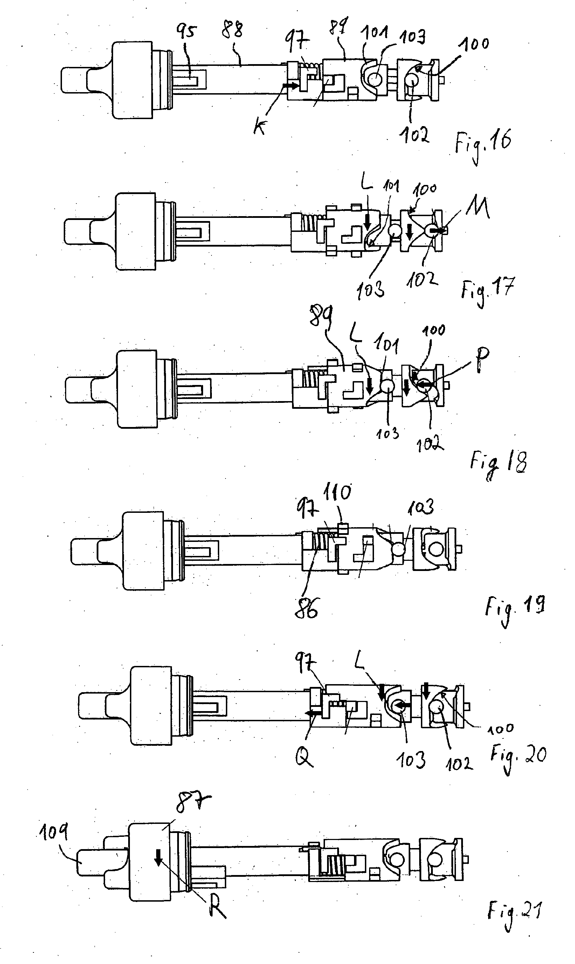

Das in Figur 1 dargestellte Stechprofil wird bei dem dargestellten Ausführungsbeispiel mittels der im folgenden anhand der Figuren 16 bis 21 erläuterten Steuereinrichtung 90 bewirkt. Die Steuereinrichtung 90 umfaßt eine Kurvensteuerung, mit der eine Drehbewegung des Antriebsrotors 89 in eine Einstich- und Rückführbewegung des Lanzettenträgers 91 umgesetzt wird.

Bei dem dargestellten Ausführungsbeispiel trägt der Antriebsrotor 89 eine erste Steuerkurve 100 und eine zweite Steuerkurve 101. Die Steuerkurven 100, 101 werden jeweils von ersten und zweiten Steuerkurvenreitern 102, 103 abgefahren, die durch eine Drehbewegung des Antriebsrotors 89 in Einstichrichtung bewegt werden. Prinzipiell würde jeweils ein erster und ein zweiter Steuerkurvenreiter 102, 103 ausreichen. Zur Vermeidung von Kippmomenten sind jedoch jeweils zwei erste Steuerkurvenreiter 102 und zwei zweite Steuerkurvenreiter 103 vorgesehen. Die Steuerkurvenreiter 102, 103 sind bei dem dargestellten Ausführungsbeispiel als Zapfen ausgebildet, die in passende Ausnehmungen 104 des Lanzettenträgers 91 eingesetzt sind. Die Steuerkurvenreiter 102, 103 können jedoch auch einstückig mit dem Lanzettenträger 91 ausgebildet sein.

In Figur 16 ist die Position der Steuerkurvenreiter 102, 104 relativ zu dem Antriebsrotor 89 zu Beginn der Vortriebsphase dargestellt. Mittels der Druckfeder 105, die sich an dem Gehäuse 84 abstützt, wird der Lanzettenträger 92 mit den Steuerkurvenreitern 102, 103 gegen den Antriebsrotor 89 gedrückt. Zu Beginn der Vortriebsphase liegen die ersten Steuerkurvenreiter 102 an der ersten Steuerkurve 100 an. Bedingt durch den Abstand zwischen der ersten Steuerkurve 100 und der zweiten Steuerkurve 101 liegen dabei in der in Figur 1 dargestellten Anfangsposition die zweiten Steuerkurvenreiter 103 nicht an der zweiten Steuerkurve 101 an. Zum Auslösen einer Einstichbewegung wird das Betätigungselement 97 in Richtung des Pfeils K verschoben, so daß sich die Sperreinrichtung 96 löst und sich der Antriebsrotor 89 zu drehen beginnt.

Während der Vortriebsphase V dreht sich der Antriebsrotor 89 in Richtung der in Figur 17 dargestellten Pfeile L. Dies bewirkt, daß die ersten Steuerkurvenreiter 102 durch die ansteigende Flanke der ersten Steuerkurve 100 in der Einstichrichtung, die durch den Pfeil M dargestellt ist, bewegt werden. Da die Steuerkurvenreiter 102 und 103 fest mit dem Lanzettenträger 91 verbunden sind, vergrößert sich bei dieser Bewegung der Abstand zwischen den zweiten Steuerkurvenreitern 103 und der zweiten Steuerkurve 101. In Figur 17 ist die Position der Steuerkurvenreiter 102, 103 am Ende der Vortriebsphase V dargestellt. In dieser Position hat der Lanzettenträger 91 den maximalen Hub erreicht, so daß ein an ihm befestigtes Stechelement 93 mit der eingestellten Einstechtiefe in die Haut eines Patienten hineinragt.

Während der in Figur 18 dargestellten Rückzugsphase R1 bewirkt die absteigende Flanke der ersten Steuerkurve 100, daß der erste Steuerkurvenreiter 102 und damit auch der mit ihm verbundene Lanzettenträger 91 eine Rückzugsbewegung in Richtung des Pfeils P ausführt. Diese Rückzugsbewegung wird gestoppt, sobald dabei der zweite Steuerkurvenreiter 103, wie in Figur 8 dargestellt, mit der zweiten Steuerkurve 101 in Kontakt kommt.

Bei der weiteren Drehung des Antriebsrotors 89 in Richtung des Pfeils L wird die Bewegung des Lanzettenträgers 91 durch die zweite Steuerkurve 101 vorgegeben, da diese in dem betreffenden Drehwinkelbereich einen im wesentlichen flachen Verlauf hat, während die erste Steuerkurve 100 weiter abfällt. Wie in Figur 19 dargestellt ist, löst sich deshalb der erste Steuerkurvenreiter 102 von der abfallenden Flanke der ersten Steuerkurve 100. In diesem Zustand wird die Drehbewegung des Antriebsrotors 89 durch eine Rückführsperre 110 gestoppt, die bei dem dargestellten Ausführungsbeispiel als ein an dem Antriebsrotor 89 angeordneter Vorsprung ausgebildet ist, der gegen den Triggerschieber 97 prallt. Auf diese Weise wird die Bewegung des Lanzettenträgers 91 für die Sammelphase gestoppt.

Die Sammelphase wird nach kurzer Zeit, beispielsweise nach ein bis zwei Sekunden beendet, indem die Rückführsperre 110 gelöst wird. Dazu wird der Triggerschieber 97 in Richtung des Pfeils Q zurückgeschoben, so daß der Antriebsrotor 89 von der Restspannung der Antriebsfeder 86 erneut in Richtung des Pfeils L in Bewegung gesetzt wird. Bei dieser zweiten Rückzugsphase liegt der zweite Steuerkurvenreiter 103 an einer abfallenden Flanke der zweiten Steuerkurve 101 an, bis der erste Steuerkurvenreiter 102 auf die erste Steuerkurve 100 auftrifft. Auf diese Weise gelangen der Lanzettenträger 91 und damit auch die ersten und zweiten Steuerkurvenreiter 102, 103 zurück in die in Figur 16 dargestellte Ausgangsposition. Anschließend kann die Antriebsfeder 86 durch Drehen des Antriebsknopfes 87 in Richtung des Pfeils R erneut gespannt werden.

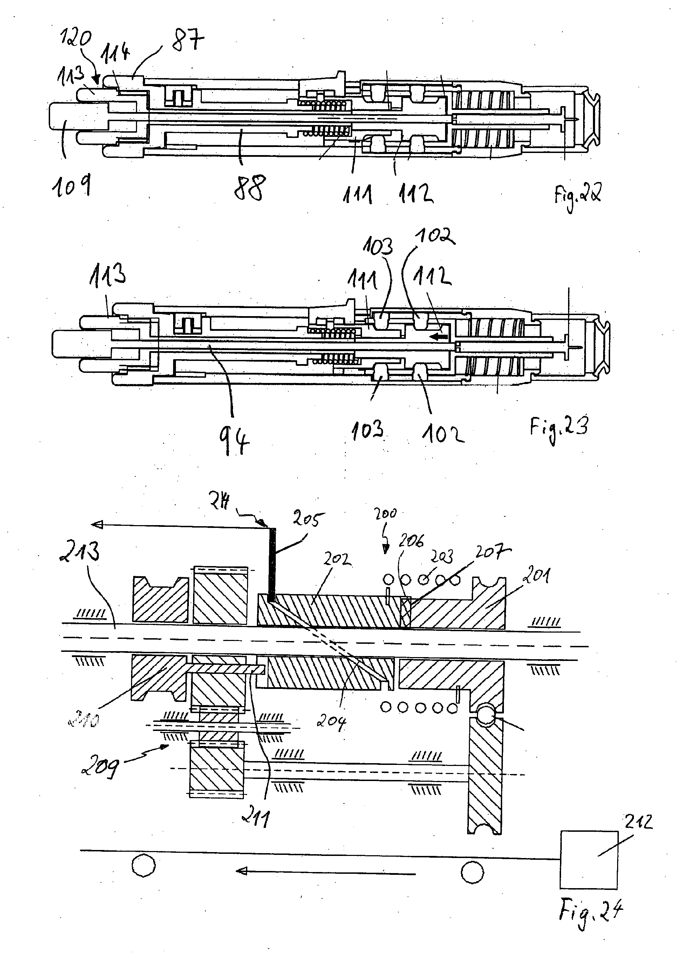

Die Einstechtiefe hängt bei der beschriebenen Steuereinrichtung von dem Abstand zwischen der ersten Steuerkurve 100 und der zweiten Steuerkurve 101 ab. Die zweite Steuerkurve 101 ist deshalb auf einem ersten Teil 111 des Antriebsrotors 89 angeordnet, der gegenüber einem zweiten Teil 112 des Antriebsrotors 89 verschoben werden kann. Der zweite Teil 112 trägt die zweite Steuerkurve 101. Um die beiden Teile 111, 112 des Antriebsrotors 89 gegen einander zu verschieben, ist eine Einstelleinrichtung 120 mit einer Einstellschraube 113 vorgesehen. Durch Drehen der Einstellschraube 113 in dem Gewinde 114 läßt sich die Position des ersten Teils 111 des Antriebsrotors 89 gegenüber dem zweiten Teil 112 des Antriebsrotors 89 in Einstichrichtung verstellen. Zur Veranschaulichung ist in Figur 23 ein Längsschnitt des Stechgeräts 81 bei maximaler Einstellung der Einstechtiefe und in Figur 23 ein Querschnitt des Stechgeräts 81 bei minimaler Einstellung der Einstechtiefe dargestellt.

Um einem Benutzer das Spannen der Antriebsfeder 86 zu erleichtern, kann das beschriebene Gerät 81 auch mit einem Elektromotor ausgerüstet werden. In diesem Fall kann auf den Spannknopf 87 des Spannrotors 88 verzichtet werden, da der Spannrotor 88 zum Spannen der Antriebsfeder 86 mittels des Elektromotors gedreht wird. Ebenso kann die Sammelphase automatisch beendet werden, indem mittels einer Zeitschaltung und einer Magnetspule oder eines anderen elektromechanischem Betätigungselements die Rückführsperre 110 am Ende der Sammelphase gelöst wird.

Bei dem dargestellten Ausführungsbeispiel wird ein 180°-Antrieb verwendet, bei dem der gesamte Ablauf mit einer halben Umdrehung der Rotoren gesteuert wird. Entsprechend sind die Steuerzapfen 102 und 103 jeweils zweifach vorhanden. Alternativ kann der Ablauf auch mit einer vollen Umdrehung gesteuert werden, wobei dann nur je ein Steuerzapfen 102 und 103 verwendet würde. Selbstverständlich besteht auch die Möglichkeit, mit je drei Steuerzapfen 102 und 103 und einer Steuerung mittels einer 120°-Drehung, jeweils mit entsprechend angepaßten Steuerkurven, zu arbeiten.

Ein weiteres Ausführungsbeispiel eines Federantriebs 200 und einer Steuereinrichtung, mit der sich das in Figur 1 dargestellte Stechprofil verwirklichen läßt, ist in Figur 24 in einer Querschnittsansicht dargestellt. Zur Vereinfachung sind dabei die übrigen Teile des Stechgeräts, insbesondere das Gehäuse, Bedienungselemente und Lanzettenträger, nicht dargestellt.

Ähnlich wie bei dem anhand der Figuren 14 bis 23 beschriebenen Ausführungsbeispiel umfaßt der Federantrieb 200 des in Figur 24 dargestellten Ausführungsbeispiels einen Spannrotor 201 und einen Antriebsrotor 202, der über eine Antriebsfeder 203 mit dem Spannrotor 201 gekoppelt ist. Der Antriebsrotor 202 trägt eine Steuerkurve 204 in Form einer Nut, die bei einer Einstich- und Rückführbewegung von einem Steuerkurvenreiter 205 abgefahren wird. Der Steuerkurvenreiter 205 ist mit einem nicht dargestellten Lanzettenträger verbunden, so daß eine Drehbewegung des Antriebsrotors 202 in einer Einstich und Rückführbewegung eines Stechelements umgesetzt werden kann.

Der Antriebsrotor 202 und der Spannrotor 201 weisen Anschlagteile 206, 207 auf, die in der in Figur 24 dargestellten Anfangsposition aneinander anschlagen. Die Anschlagteile 206, 207 dienen dazu, eine Vorspannung der Antriebsfeder 208 aufzunehmen und am Ende der Rückzugsphase R1 eine Drehbewegung des Antriebsrotors 202 zu stoppen.

In der in Figur 24 dargestellten Ausgangsposition befindet sich der Steuerkurvenreiter 205 in einer Extremalposition, die mit einem maximalen Hub eines nicht dargestellten Lanzettenträgers verbunden ist. Zum Einstellen der Einstechtiefe wird der Antriebsrotor 202 mit der Einstelleinrichtung 209, die beispielsweise einen motorisch getriebene Schnecke 215 umfaßt, aus der Ausgangsposition gegenüber dem Spannrotor 201 um einen Drehwinkel $\alpha$ verdreht, so daß der Steuerkurvenreiter 205 in der Steuerkurve 204 wandert und ein an dem Lanzettenträger befestigtes Stechelement (nicht dargestellt) um eine dem Drehwinkel $\alpha$ entsprechende Strecke zurückgezogen wird.

Der Drehwinkel $\alpha$ wird so gewählt, daß der Steuerkurvenreiter 205 um die gewünschte Einstechtiefe dm plus eine konstante Anlaufstrecke zurückgezogen wird. Hat die Steuerkurve 204 wie bei dem dargestellten Ausführungsbeispiel eine konstante Steigung, ist der Drehwinkel $\alpha$ die Summe eines Drehwinkels $\alpha1$, der einen Lanzettenhub in Höhe der gewünschten Einstechtiefe bewirkt, und eines Drehwinkels $\alpha2$, der einen dem Anlaufweg der Lanzette entsprechenden Lanzettenhub bewirkt. Sobald der Steuerkurvenreiter 205 die gewünschte Position erreicht hat, wird der Antriebsrotor 202 in der erreichten Position arretiert. Hierfür kann ein Anfangsanschlag 211, über den der Antriebsrotor 202 in die gewünschte Position gedreht wurde, verwendet werden.

Gleichzeitig oder anschließend an die Drehung des Antriebsrotors 202 wird der Spannrotor 201 in eine entgegengesetzte Richtung gedreht. Dabei wird der Antriebsrotor gegenüber der in Figur 22 dargestellten Ausgangsposition um einen Drehwinkel $\alpha-\alpha2-\beta=\alpha1-\beta$ verdreht, wobei der Drehwinkel $\beta$ einem Lanzettenhub in Höhe der gewünschten Reststechtiefe entspricht. In der sich ergebenden Drehwinkelstellung wird der Spannrotor 201 dann arretiert.

Der letzte Schritt zum Einstellen der Einstechtiefe besteht darin, daß der gesamte Antrieb 200 mit einem Elektromotor 212 entlang der Führung 213 in Einstichrichtung verschoben wird, bis der Abstand zwischen der Spitze des Stechelements und der Hautoberfläche der Anlaufstrecke entspricht. Sobald diese Position erreicht ist, kann ein Einstich eingeleitet werden, indem die Arretierung 211 des Antriebsrotors 202 gelöst wird. Daraufhin dreht sich der Antriebsrotor 202 bis seine Drehbewegung durch aneinanderstoßen der Anschlagteile 206, 207 des Antriebsrotors 202 und des Spannrotors 201 gestoppt wird.

Während der Vortriebsphase V dreht sich der Antriebsrotor 202 um den Drehwinkel $\alpha$ zurück in die in Figur 24 dargestellte Position. Dies bewirkt über den Steuerkurvenreiter 205 einen Hub des Stechelements in Höhe der gewünschten Einstechtiefe plus der Anlaufstrecke, die dem Abstand zwischen der Hautoberfläche und dem Stechelement beim Auslösen der Einstichbewegung entspricht. Bei der anschließenden Rückzugsphase R1 durchläuft der Antriebsrotor 202 einen Drehwinkelbereich $\alpha-\alpha2-\beta=\alpha1-\beta$, so daß die dadurch bewirkte Rückzugsbewegung des Stechelements dazu führt, daß das Stechelement mit der vorgegebenen Reststechtiefe in der Haut stecken bleibt, sobald der Antriebsrotor 202 durch die Anschlagteile 206, 207 gestoppt und dadurch die Sämmelphase eingeleitet wird.

Durch Einstellen der Positionen des Antriebsrotors 202 und des Spannrotors 201 vor dem Auslösen eines Einstichs wird ein aktiver Abschnitt:der Steuerkurve 204 ausgewählt, der während der Vortriebsphase V und der Rückzugsphase R1 von dem Steuerkurvenreiter 2.05 abgefahren wird. Der Anfang des aktiven Abschnitts definiert dabei die Einstechtiefe, das Ende des aktiven Abschnitts die Reststechtiefe. Vor dem aktiven Abschnitt liegt ein inaktiver Abschnitt, der um so kleiner ist, je größer die eingestellte Einstechtiefe ist. Hinter dem aktiven Abschnitt liegt ein zweiter inaktiver Abschnitt, der um so kleiner ist, desto kleiner die eingestellte Reststechtiefe ist. Der Steuerkurvenreiter 205, die Steuerkurve 204, die Anschlagteile 206, 205 und der Anfangsanschlag 211 bilden also eine Steuereinrichtung 214 zum Steuern der Einstich- und Rückführbewegung.

Der Abstand zwischen der Spitze des Stechelements und der Hautoberfläche kann beispielsweise durch eine Widerstandsmessung und/oder kapazitive und/oder induktive Messung, bestimmt werden, bei der bevorzugt die Spitze des Stechelements als Elektrode verwendet wird.

Eine weitere Möglichkeit, die Einstechtiefe einzustellen besteht darin, den Antriebsrotor 202 stets um den selben Winkel $\alpha$ aus der in Figur 24 dargestellten Ausgangsposition zu verdrehen, also stets in der selben Position zu arretieren. Diese wird bevorzugt so gewählt, daß der Steuerkurvenreiter 205 so weit wie möglich zurückgezogen wird. In diesem Fall wird zunächst die Position der Hautoberfläche in Relation zu einem festen Referenzpunkt des Gerätegehäuses bestimmt und der Antrieb 200 anschließend soweit in Einstichrichtung verfahren, daß der bei der Vortriebsphase auftretenden Lanzettenhub die gewünschte Einstechtiefe ergibt.

Nach dem Ende der Sammelphase wird das Stechelement vollständig aus der Haut herausgezogen, indem der Spannrotor 201 in der entsprechenden Richtung gedreht wird. Wegen der unter Vorspannung stehenden Antriebsfeder 203 folgt der Antriebsrotor 202 dabei der Drehbewegung des Spannrotors 201, so daß der Steuerkurvenreiter 205 den verbleibenden Abschnitt der Steuerkurve 204 abfährt und auf diese Weise eine Rückführbewegung des Stechelements bewirkt. Alternativ kann auch ein Linearantrieb für die Vorabdetektion der Position der Hautoberfläche und für die Rückzugsphase R2 verwendet werden.

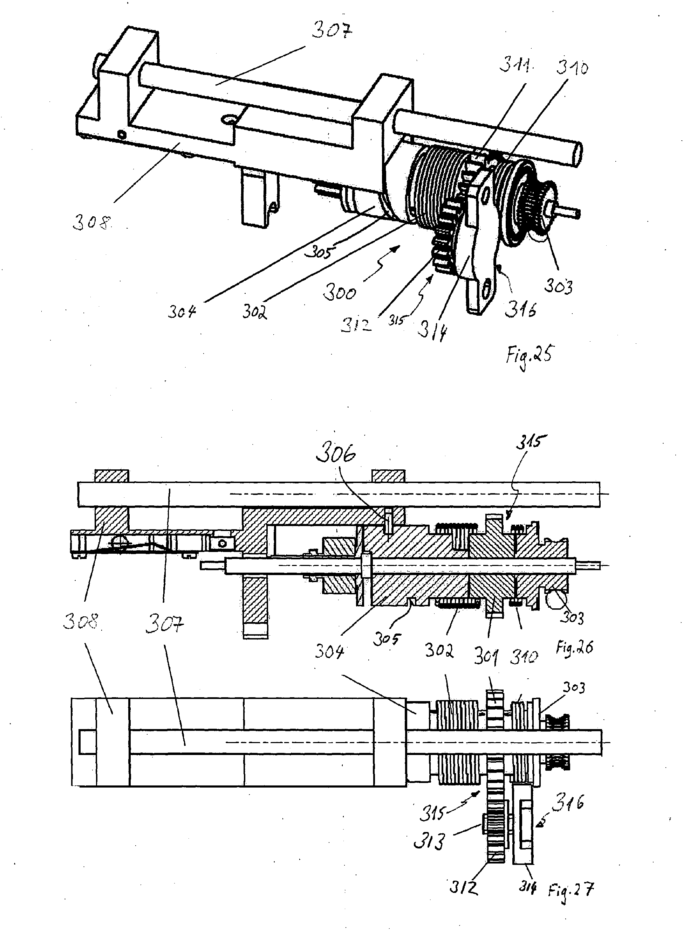

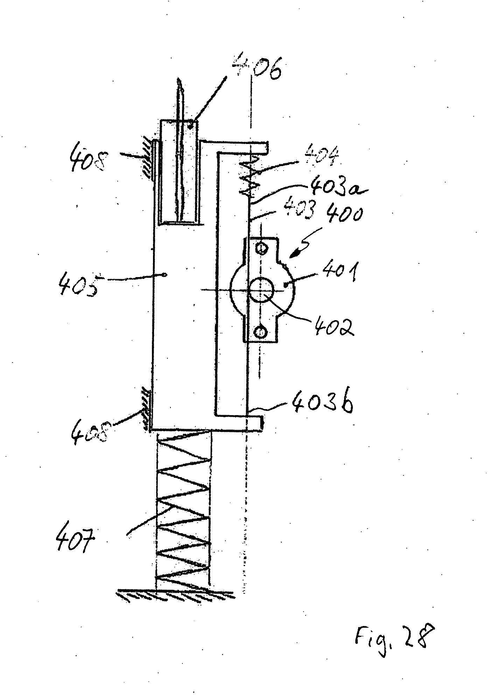

Bei einem abrupten Abstoppen des Stechelements am Ende der Rückzugsphase können unter Umständen schmerzhafte Vibrationen auftreten. Bevorzugt wird deshalb zum Abbremsen der Rückzugsbewegung ein Dämpfer eingesetzt. Ein Ausführungsbeispiel eines Antriebs 300, der mit einem Dämpfungsmechanismus 315 gekoppelt ist, ist in Figur 25 in einer Schrägansicht, in Figur 26 in einer Querschnittsansicht und in Figur 27 in einer Seitenansicht dargestellt. Der Dämpfungsmechanismus 315 umfaßt einen Dämpfungsrotor 301, der über Zahnkränze 311, 312 mit einem Rotationsdämpfer 316 gekoppelt ist, der von einem Rotationskörper 313 und einem mit Dämpfungsflüssigkeit gefüllten Gehäuse 314 gebildet wird.

Der dargestellte Antrieb 300 umfaßt ebenso wie der anhand von Figur 24 beschriebene Antrieb 200 eine Antriebsfeder 302, einen Spannrotor 303 zum Spannen der Antriebsfeder 302 und einen von der Antriebsfeder 302 angetriebenen Antriebsrotor 304. Der Antriebsrotor 304 trägt eine Steuerkurve 305, die bei einer Einstichbewegung von einem Steuerkurvenreiter 306 einer Kurvensteuerung abgefahren wird und eine Einstich- und Rückführbewegung eines Lanzettenträgers 307 bewirkt, der in Einstichrichtung beweglich von einer Führung 308 gehalten wird.

Der Antrieb 300 umfaßt zusätzlich den Dämpfungsrotor 301, der zwischen dem Antriebsrotor 304 und dem Spannrotor 303 angeordnet ist. Der Spannrotor 303, der Dämpfungsrotor 301 und der Antriebsrotor 305 weisen ebenso wie die Rotoren 201, 202 des anhand des von Figur 24 erläuterten Ausführungsbeispiels Anschlagteile auf, mit denen benachbarte Rotoren aneinander anliegen und welche die Vorspannung der Antriebsfeder 302 aufnehmen. Diese Anschlagteile sind in den Figuren 23 bis 25 nicht dargestellt, entsprechen in ihrer Ausgestaltung jedoch den in Figur 24 dargestellten Anschlagteilen 206 und 207.

Der Antrieb 300 umfaßt zusätzlich eine Dämpfungsfeder 310, die zwischen dem Spannrotor 303 und den Dämpfungsrotor 301 wirkt. Die Vorspannung der Dämpfungsfeder 310 ist geringer als die Vorspannung der Antriebsfeder 302 und wirkt der Kraft der Antriebsfeder 302 entgegen. Die Federkraft der Dämpfungsfeder 310 wirkt also darauf hin, daß sich die Anschlagteile des Zwischenrotors 301 und des Spannrotors 303 durch eine relative Drehbewegung der beiden Rotoren 301, 303 voneinander entfernen, jedoch wird dies durch die stärkere Federkraft der Antriebsfeder 302 verhindert.

Der Dämpfungsrotor 301 trägt an seiner Außenseite einen Zahnkranz 311, der über einen Zahnkranz 312 an eine Dämpferwelle 313 gekoppelt ist, die sich in dem Gehäuse 314 drehen kann, das mit einer viskosen Flüssigkeit gefüllt ist und auf diese Weise ein Dämpfungslager bildet.

Die Einstechtiefe und die Reststechtiefe können bei dem in den Figuren 25 bis 27 dargestellten Ausführungsbeispiel in gleicher Weise wie bei dem anhand von Figur 24 beschriebenen Ausführungsbeispiel eingestellt werden. Der einzige Unterschied besteht darin, daß der Antriebsrotor 305 am Ende der Rückzugsphase R1 nicht unmittelbar durch ein Anschlagteil des Spannrotors 303 abgestoppt wird, sondern mit seinem Anschlagteil gegen ein Anschlagteil des Dämpfungsrotors 301 prallt. Zur Vermeidung von Wiederholungen wird deshalb im folgendem der Bewegungsablauf der einzelnen Rotoren nicht im Hinblick auf die Reststechtiefe sondern lediglich auf durch den Dämpfungsrotor 301 bedingte Besonderheiten erläutert.

Beim Spannen der Antriebsfeder 302 wird der Spannrotor 303 gegenüber dem Zwischenrotor 301 verdreht. Dies führt dazu, daß die Anschlagteile des Spannrotors 303 und des Dämpfungsrotors 301 sowie des Antriebsrotors 304 und des Dämpfungsrotors 301 voneinander abheben. Am Ende des Spannvorgangs ist der Dämpfungsrotor 301 wegen der Dämpfungsfeder 310 gegenüber dem Spannrotor 303 um einen Winkel $\chi$ verdreht.

Wird ein Einstich ausgelöst, dreht sich der Antriebsrotor 304, so daß ein Lanzettenträger 307 durch die Kurvensteuerung 305, 306 gemäß dem vorhergehenden Ausführungsbeispiel eine Einstich- und Rückführbewegung ausführt. Am Ende der Rückzugsphase schlagen die Anschlagteile des Antriebsrotors 304 des Dämpfungsrotors 301 aufeinander, so daß der Dämpfungsrotor 301 ebenfalls in Drehung versetzt wird. Dieser Drehbewegung wirkt die Spannung der Dämpfungsfeder 310 entgegen. Zusätzlich wird der Dämpfungsrotor 301 über den Zahnkranz 312 und die in dem Dämpfungslager 314 gelagerte Welle 313 gebremst.

Am Ende der Rückzugsphase R1 setzen die Anschlagteile des Dämpfungsrotors 301 und des Spannrotors 303 wieder aufeinander auf, so daß die Drehbewegung des Antriebsrotors 304 und damit auch die Rückführbewegung des Lanzettenträgers 307 gestoppt wird. Um die Reststechtiefe präzise zu definieren wird der Spannrotor 303 arretiert, beispielsweise indem er mit einer Sperrklinke gegen das Gerätegehäuse (nicht dargestellt) verrastet ist oder indem er mit einem selbsthemmenden Getriebe in Eingriff steht.

Anstelle einer Zahnradkopplung 311, 312 des Dämpfungsrotors 301 mit der in dem Dämpfungslager 314 geführten Welle 313 kann ein Dämpfer'auch in den Dämpfungsrotor 301 und den Spannrotor 303 integriert werden, um die Drehbewegung des Antriebsrotors 304 am Ende der Rückzugsphase R1 langsam abzustoppen. Das Dämpfungslager 314 oder auch ein in dem Dämpfungsrotor 301 integrierter Dämpfer kann beispielsweise als handelsüblicher Rotationsdämpfer ausgebildet sein. Üblicherweise wird eine Dämpfungswirkung dabei dadurch erzielt, daß ein Rotationskörper in einer Füllung aus zähem Öl, beispielsweise Silikonöl rotiert, das von einer Gehäusewand eingeschlossen ist. Die Stärke der Dämpfungswirkung hängt dabei von der Ölviskosität und dem Abstand des Rotationskörpers, beispielsweise der Welle 313, von der Gehäusewand des Dämpfers, beispielsweise des Lagers 314, ab. Wird der Rotationskörper dabei als ein Stapel von flachen Scheiben ausgebildet, die jeweils einen festen Abstand zueinander haben, und fügt man in das Gehäuse eine korrespondierende Anordnung von Platten ein, die sich in die Zwischenräume zwischen den rotierenden Scheiben einschwenken lassen, kann man die Dämpfercharakteristik durch mehr oder weniger starkes ineinandergreifen der Platten und der Scheiben verändern.

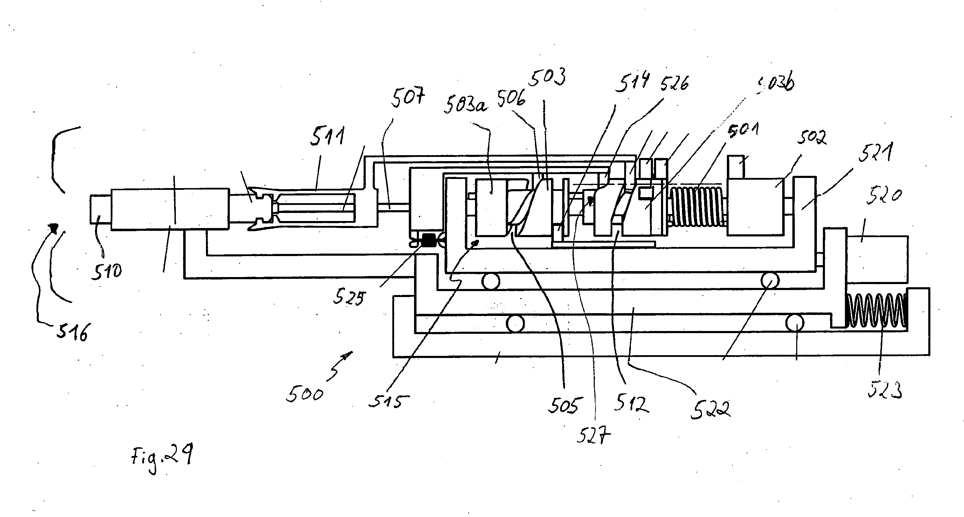

In Figur 28 in ein weiteres Ausführungsbeispiel eines Dämpfungsmechanismus dargestellt, der beispielsweise bei dem anhand der Figuren 4 und 5 erläuterten Ausführungsbeispiel eingesetzt werden kann, um ein abruptes Abstoppen am Ende der Rückzugsbewegung durch eine Dämpfung zu Vermeiden. Der Dämpfungsmechanismus 400 umfaßt einen Rotationsdämpfer 401 mit einem Rotationskörper 402 in Form einer Welle, die über einen Faden 403 und eine Feder 404 mit einem Lanzettenträger 405 gekoppelt ist. Der Faden 403 ist um die Welle 402 gewickelt und mit einem Ende 403a fest mit dem Lanzettenträger 405 verbunden, während sein anderes Ende 403b über die Feder 404 mit dem Lanzettenträger 405 gekoppelt ist. Befindet sich der Lanzettenträger 405 in Ruhe, beispielsweise vor dem Auslösen einer Einstichbewegung, wird der Faden 403 von der Feder 404 straff gespannt.

Bei einer Einstichbewegung wird der Lanzettenträger 405 und damit auch die in ihn eingesetzte Probenentnahmeeinheit 406 mittels einer Antriebsfeder 407 in Einstichrichtung auf einem durch Linearführungen 408 vorgegebenen Einstichweg beschleunigt. Dies hat zur Folge, daß das hintere Ende 403b des Fadens 403 entlastet wird, während die Feder 404 durch die Einstichbewegung des Lanzettenträgers 405 weiter gespannt wird. Die von der Feder 404 auf den Faden 403 ausgeübte Federkraft steigt deshalb an, so daß der Faden 403 relativ zu der Welle 402 bewegt wird. Da dabei auf das hintere Ende des Fadens 403b von dem Lanzettenträger 405 keine Kraft ausgeübt wird, kann der Faden mit einer geringen Reibungskraft auf der Welle 402 gleiten, so daß die Vortriebsbewegung des Lanzettenträgers 405 durch den Rotationsdämpfer 401 nur geringfügig gebremst wird.

Bei einer anschließenden Rückzugsphase, während der die Antriebsfeder 407 bei geeigneter Dimensionierung eine Zugkraft auf' den Lanzettenträger 405 ausübt, wird auf das hintere Ende 403b des Fadens 403 von dem Lanzettenträger 405 eine Zugkraft ausgeübt. Da das vordere Ende 403a des Fadens 403 von der Feder 404 straff gespannt wird bewirkt dies, daß der Faden 403 mit seinen Umschlingungen straff an der Welle 402 anliegt. Bei der Rückzugsbewegung herrscht deshalb zwischen dem Faden 403 und der Welle 402 eine wesentlich höhere Reibungskraft als während der Vortriebsphase. Deshalb kann der Faden 403 bei der Rückzugsphase nicht oder nur in geringem Maß auf der Welle 402 gleiten. Die von dem Lanzettenträger 405 auf das hintere Ende 403b des Fadens 403 ausgeübte Zugkraft wird deshalb durch Reibungskräfte auf die Welle 402 übertragen und bewirkt ein Drehmoment, durch das die Welle 402 in Drehung versetzt wird. Die Welle 402 bildet den Rotationskörper des Rotationsdämpfers 400 und ist in der Kammer 401 in einer Dämpfungsflüssigkeit, beispielsweise einem zähem Öl, drehbar gelagert. Auf diese Weise kann von dem Rotationsdämpfer 400 bei der Rückführbewegung die kinetische Energie des Lanzettenträgers 405 aufgenommen und dissipiert werden, so daß sich eine gedämpfte Rückführbewegung mit einem langsamen abstoppen ergibt.

Anstatt den Dämpfungsmechanismus an den Lanzettenträger 405 zu koppeln, kann man ihn beispielsweise bei den anhand der Figuren 4 und 5 beschriebenen Ausführungsbeispiel auch an das als Hülse ausgebildete Referenzelement 35 ankoppeln. Wird nämlich bei der Rückzugsphase von dem Lanzettenträger auch das Referenzelement zurückgezogen, kann die Bewegung des Lanzettenträgers auch durch eine an dem Referenzelement ansetzende Dämpfung gebremst werden. Wird das Referenzelement bei einem Einstich bereits von dem Benutzer an die Hautoberfläche angesetzt, ist eine Freilauffunktion des Dämpfungsmechanismus, die bei den anhand von Figur 28 beschriebenen Ausführungsbeispiel dadurch erreicht wird, daß der Faden 403 die Welle 402 umschlingt, verzichtet werden. In einem derartigem Fall genügt es, das Referenzelement beispielsweise mittels einer Zahnstange an die den Rotationskörper bildende Welle 402 des Rotationsdämpfers, oder direkt an einen entsprechenden Lineardämpfer, zu koppeln.

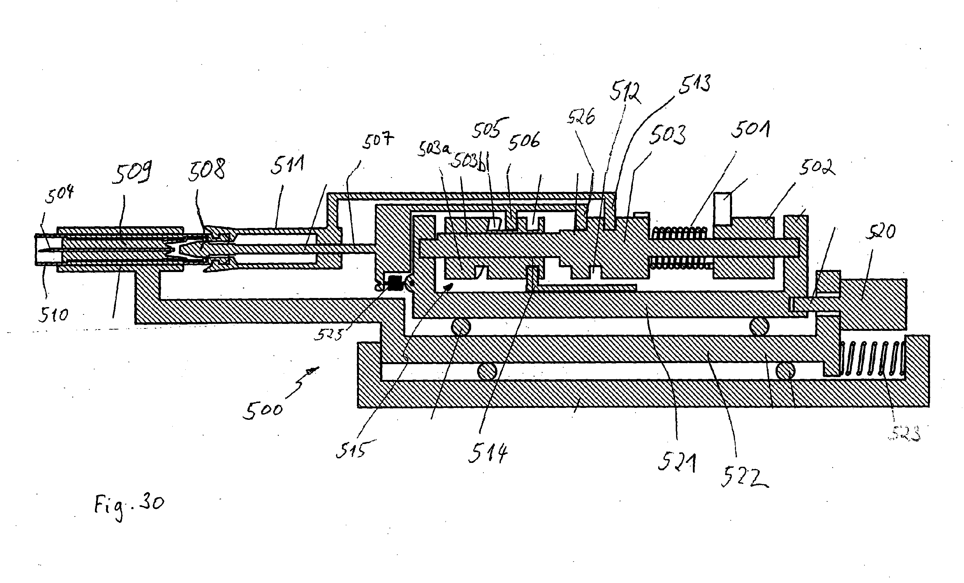

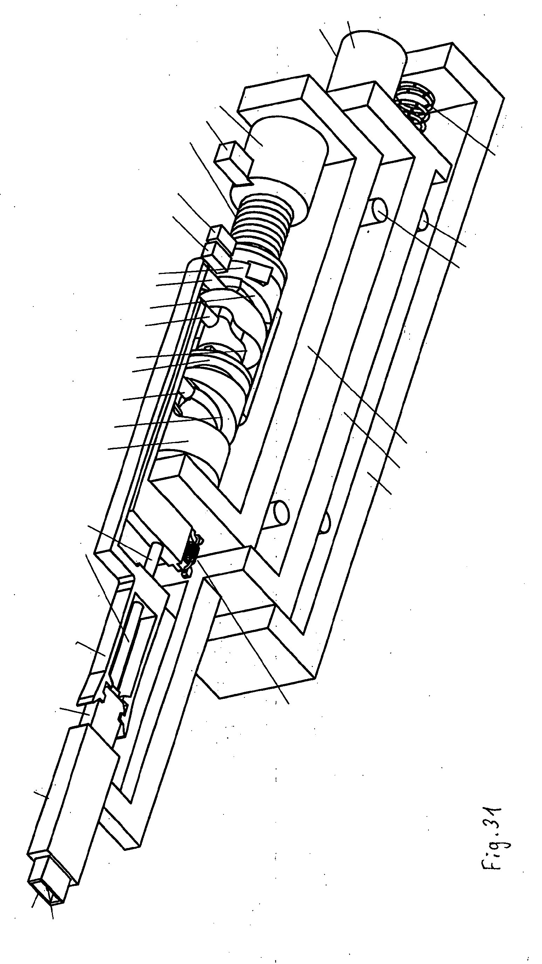

Ein weiteres Ausführungsbeispiel eines Stechsystems 500, mit dem sich das in Figur 1 dargestellte Stechprofil verwirklichen läßt, ist in Figur 29 in einer Seitenansicht, in Figur 30 in einer Querschnittsansicht und in Figur 31 in einer Schrägansicht dargestellt. Zu dem Antrieb 515 des dargestellten Stechgeräts gehört eine Antriebsfeder 501, ein Spannrotor 502 zum Spannen der Antriebsfeder und ein von der Antriebsfeder 501 angetriebener Antriebsrotor 503. Zum Umsetzen einer Drehbewegung des Antriebsrotors 503 in eine Einstich- und Rückführbewegung eines Stechelements 504 dient eine Steuereinrichtung in Form einer Kurvensteuerung. Eine Lanzetten-Steuerkurve 505 ist als Nut in dem Antriebsrotor 503 ausgebildet und wird von einem Lanzetten-Steuerkurvenreiter 506 in Form eines in die Steuerkurve 505 eingreifenden Zapfens abgefahren, der mit einem Lanzettenträger 507 verbunden ist. Der Lanzettenträger 507 hat einen Kopf 508, der formschlüssig mit einer Probenentnahmeeinheit 509, zu der das Stechelement 504 gehört, verrastet.

Ebenso wie bei den anhand der Figuren 4 und 5 beschriebenen Ausführungsbeispiel wird die Probenentnahmeeinheit 509 zusammen mit einem Referenzelement 510 in Form einer Hülse in das Stechgerät eingelegt. Bei einem Einstich liegt das Referenzelement 510 an der Haut eines Benutzers an und sorgt so für einen Referenzpunkt zur Definition der Einstechtiefe.

Das Referenzelement 510 verrastet mit einem Referenzelementträger 511, dessen Bewegung durch eine Kurvensteuerung mit einer Referenz-Steuerkurve 512 und einem Referenz-Steuerkurvenreiter 513 gesteuert wird. Die Steuerkurve 512 ist ebenfalls als Nut in dem Antriebsrotor 503 angeordnet, in die der Steuerkurvenreiter 513 des Referenzelementträgers 511 eingreift. Zum Einstellen der Einstechtiefe ist der Abstand der beiden Steuerkurven 505, 512 mittels einer Einstelleinrichtung 514 in Form einer justierbaren Axiallagerung einstellbar. Ähnlich wie bei dem anhand der Figur 14 erläuterten Ausführungsbeispiel sind die beiden Steuerkurven 505, 512 auf einem ersten Teil 503a des Antriebsrotors 503 bzw. auf einem zweiten Teil 503b des Antriebsrotors 503 angeordnet. Mittels der Einstelleinrichtung 514 kann der Abstand zwischen dem ersten Teil 503a des Antriebsrotors 503 und dem zweiten Teil 503b des Antriebsrotors 503 variiert werden.

Für einen Einstich wird das Stechgerät mit einer nur in Figur 29 schematisch angedeuteten Gehäuseöffnung 516 an eine Hautoberfläche des Benutzers angedrückt und der Abstand des Referenzelements 510 von der Hautoberfläche festgelegt. Dazu wird mittels eines Elektromotors 520 ein in Einstichrichtung verschiebbarer erster Schlitten 521, auf dem der Antrieb 515 und damit auch der Lanzettenträger 507 und der Referenzelementträger 511 gelagert sind, an die Hautoberfläche herangefahren, bis das Referenzelement 510 die Haut berührt. Dies kann elektronisch, beispielsweise durch eine induktive oder kapazitive Messung, festgestellt werden. Anschließend wird der Schlitten wieder etwas zurückgefahren, bis ein definierter Abstand zu der Hautoberfläche besteht.

Nach dem Auslösen eines Einstichs werden der Lanzettenträger 507 und der Referenzelementträger 511 während der Vortriebsphase durch eine ansteigende Flanke der entsprechenden Steuerkurve 505, 512 nach vorn geschoben. Bei dem eigentlichen Einstichvorgang, d. h. während des Eindringens des Stechelements 504 in die Hautoberfläche liegt das Referenzelement 510 an der Hautoberfläche an, so daß ein Referenzpunkt für eine präzise Einstechtiefe definiert ist. Da die elastischen Eigenschaften der Hautoberfläche von Patient zu Patient verschieden sind und auch von der Einstichstelle selbst abhängen, ist der erste Schlitten 521 auf einem zweiten Schlitten 522 gelagert, der gegen eine Andruckkontrollfeder 523 zurückgeschoben werden kann. Durch die Andruckkontrollfeder 523 wird eine maximale Andruckkraft definiert, die über das Referenzelement 510 auf die Hautoberfläche wirken kann. Höhere Andruckkräfte werden durch eine Verschiebung des zweiten Schlittens 522 gegen die Andruckkontrollfeder 523 ausgeglichen.

Nach Erreichen der maximalen Einstechtiefe werden der Lanzettenträger 507 und der Referenzelementträger 511 zurückgezogen. Die Kurvensteuerung des Lanzettenträgers 507 hat dabei die Besonderheit, daß sich der Lanzetten-Steuerkurvenreiter 506 bei der Rückführbewegung aus seinem Eingriff mit der Steuerkurve 505 löst. Dies bedeutet, daß der Steuerkurvenreiter nicht, wie sonst üblich, in der gesamten Steuerkurve so geführt ist, daß jeder Position des Steuerkurvenreiters auf der Steuerkurve eine definierte Längsposition des Reiters und damit des gesteuerten Elementes (Stechelement, Referenzelement) in Einstichrichtung entspricht. Vielmehr ist die Längsposition des Steuerkurvenreiters und damit des gesteuerten Elementes bei gelöstem Eingriff der Steuerkurve allenfalls in einer Raumrichtung (entgegen der Einstichrichtung oder in Einstichrichtung) begrenzt, mindestens in der entgegengesetzten Raumrichtung jedoch frei. Dies wird bei dem dargestellten Ausführungsbeispiel dadurch erreicht, daß die die Steuerkurve 505 bildende Nut auf der rückführenden Flanke soweit verbreitert ist, daß der Steuerkurvenreiter 506 dort nicht mehr geführt wird. Der Lanzettenträger 507 wird deshalb während der Rückzugsphase nicht aktiv von dem Lanzetten-Steuerkurvenreiter 506 zurückgezogen.

Der Lanzettenträger 506 wird statt dessen durch eine Rückstellfeder 525 zurückgezogen. Die Rückstellfeder 525 koppelt den Lanzettenträger 507 mit dem ersten Schlitten 521 und damit auch mit dem Antrieb. Bei der Rückzugsphase wird der Lanzettenträger 507 deshalb von der Rückstellfeder 525 relativ zu dem Antrieb zurückgeschoben, bis er mit einem zweiten Steuerkurvenreiter 526 an eine weitere Steuerkurve 525, die auf dem zweiten Teil 503b des Antriebsrotors 503 angebracht ist, stößt. Die weitere Steuerkurve 527 bildet also eine Rückführsperre, mit der das Stechelement 5 am Ende der Rückzugsphase R1 abgestoppt wird. Kommt der Antriebsrotor 503 am Ende der Rückzugsphase R1 zum Stehen, ist auf diese Weise eine definierte Lage des Lanzettenträgers 507 gegenüber dem Referenzelementträger 511 gegeben, bei der das Stechelement 504 mit der vorgegebenen Reststechtiefe über die Kante des Referenzelements 510 hinausragt. Nach Abschluß der Sammelphase wird das Stechelement 505 vollständig aus der Haut herausgezogen, indem der erste Schlitten 521 mit dem Elektromotor 520 zurückgefahren wird.

Das beschriebene Stechsystem hat den Vorteil, daß das Referenzelement nur kurze Zeit, vorzugsweise weniger als 2 ms, gegen die Haut gepreßt wird. Auf diese Weise kann der Einstich in seiner Tiefe kontrolliert werden, jedoch führen die viskoelastischen Eigenschaften der Haut bei einer derartig kurzen Anpressen noch nicht dazu, daß die präzise Definition der Reststechtiefe durch eine Verformung der Haut beeinträchtigt wird.

Bezugszeichenliste

| | |
|---|---|
| 1 | Steuereinrichtung |
| 2 | Schwenkelement |
| 3 | Anschlag |
| 4 | Anschlag |
| 5 | Stechelement |
| 6 | Antriebsfeder |
| 10 | Steuerelement |
| 11 | Steuerkurve |
| 12 | Steuerkurvenreiter |
| 20 | Stechgerät |
| 21 | Antriebsfeder |
| 22 | Schubstange |
| 23 | Triggerelement |
| 24 | Anschlag von 22 |
| 25 | Anschlag von 26 |
| 26 | Einstelleinrichtung |
| 27 | Einstellschraube |

| | | | | |
|---|---|---|---|---|
| 28 | Gewinde der Einstellschraube | | 101 | zweite Steuerkurve |
| 30 | Referenzfläche | | 102 | erster Steuerkurvenreiter |
| 31 | Kopf der Schubstange | | 103 | zweiter Steuerkurvenreiter |
| 32 | Kupplungsausnehmung | | 105 | Druckfeder |
| 33 | Lanzettenkörper | | 109 | Kopf der Auswurfstange |
| 34 | Lanzette | | 110 | Rückführsperre |
| 35 | Stechtiefenreferenzelement | | 111 | erster Teil des Antriebsrotors 89 |
| 37 | Sperrelement | | 112 | zweiter Teil des Antriebsrotors 89 |
| 38 | Rückführsperre | | 113 | Einstellschraube |
| 39 | Federelement | | 114 | Gewinde der Einstellschraube |
| 40 | Gleitfläche | | 120 | Einstelleinrichtung |
| 41 | Gleitfläche | | 200 | Federantrieb |
| 42 | Rastvorsprung | | 201 | Spannrotor |
| 43 | Anschlag von 42 | | 202 | Antriebsrotor |
| 44 | Anschlag von 37 | | 203 | Antriebsfeder |
| 45 | Sperrriegel | | 204 | Steuerkurve |
| 50 | Stechgerät | | 205 | Steuerkurvenreiter |
| 51 | Gehäuse | | 206 | Anschlagteil des Antriebsrotors |
| 52 | Lanzettenmagazin | | 207 | Anschlagteil des Spannrotors |
| 54 | Schlitten | | 209 | Einstelleinrichtung |
| 55 | Federantrieb | | 210 | Schnecke |
| 56 | Antriebsfeder | | 211 | Anfangsanschlag |
| 57 | Rückzugsfeder | | 212 | Elektromotor |
| 58 | Klinkenfeder | | 213 | Führung |
| 59 | Basisplatte | | 214 | Steuereinrichtung |
| 60 | Klinke | | 300 | Antrieb |
| 61 | Kopf | | 301 | Dämpfungsrotor |
| 62 | Ansatzfläche | | 302 | Antriebsfeder |
| 63 | Schlittenvorsprung | | 303 | Spannrotor |
| 64 | Rückführelement | | 304 | Antriebsrotor |
| 65 | Sperrklinke | | 305 | Steuerkurve |
| 66 | Rückführsperre | | 306 | Steuerkurvenreiter |
| 67 | Sperrklinke | | 307 | Lanzettenträger |
| 69 | Zapfen | | 308 | Führung des Lanzettenträgers |
| 70 | Gleitfläche | | 310 | Dämpfungsfeder |
| 71 | Schubfläche | | 311 | Zahnenkranz des Dämpfungsrotors |
| 72 | Betätigungsarm | | 312 | Zahnenkranz |
| 73 | Andruckfläche | | 313 | Dämpferwelle |
| 74 | Kopf | | 314 | Gehäuse |
| 75 | Gleitfläche | | 315 | Dämpfungsmechanismus |
| 76 | Stufe | | 316 | Rotationsdämpfer |
| 80 | Stechsystem | | 400 | Dämpfungsmechanismus |
| 81 | Stechgerät | | 401 | Rotationsdämpfer |
| 82 | Gehäuse | | 402 | Rotationskörper |
| 83 | Gehäuseöffnung | | 403 | Faden |
| 84 | Andruckring | | 403a | vorderes Fadenende |
| 86 | Antriebsfeder | | 403b | hinteres Fadenende |
| 87 | Spannknopf | | 404 | Feder |
| 88 | Spannrotor | | 405 | Lanzettenträger |
| 89 | Antriebsrotor | | 406 | Probenentnahmeeinheit |
| 90 | Steuereinrichtung | | 407 | Antriebsfeder |
| 91 | Lanzettenträger | | 408 | Linearführungen |
| 92 | Probenentnahmeeinheit | | 500 | Stechsystem |
| 93 | Stechelement | | 501 | Antriebsfeder |
| 94 | Auswurfstange | | 502 | Spannrotor |
| 96 | Sperreinrichtung | | 503 | Antriebsrotor |
| 97 | Auslöseelement | | 503a | erstes Teil des Antriebsrotors |
| 100 | erste Steuerkurve | | 503b | zweites Teil des Antriebsrotors |

| 504 | Stechelement |
| 505 | Lanzetten-Steuerkurve |
| 506 | Lanzetten-Steuerkurvenreiter |
| 507 | Lanzettenträger |
| 508 | Kopf des Lanzettenträgers |
| 509 | Probenentnahmeeinheit |
| 510 | Referenzelement |
| 511 | Referenzelementträger |
| 512 | Referenz-Steuerkurve |
| 513 | Referenz-Steuerkurvenreiter |
| 514 | Einstelleinrichtung |
| 515 | Antrieb |
| 516 | Gehäuseöffnung |
| 520 | Elektromotor |
| 521 | erster Schlitten |
| 522 | zweiter Schlitten |
| 523 | Andruckkontrollfeder |
| 525 | Rückstellfeder |
| 526 | zweiter Steuerkurvenreiter |
| 527 | weitere Steuerkurve |

| A | Stechtiefe bei Beginn des Abbremsens des Stechelements |
| dm | Einstechtiefe |
| dr | Reststechtiefe |
| $\Delta$d | Rückzugsstrecke |
| V | Vortriebsphase |
| R1 | Rückzugsphase |
| S | Sammelphase |
| R2 | zweite Rückzugsphase |
| F | Pfeilrichtung |
| H | Pfeilrichtung |
| K | Pfeilrichtung |
| L | Pfeilrichtung |
| M | Pfeilrichtung |
| P | Pfeilrichtung |
| Q | Pfeilrichtung |
| $\alpha$ | Gesamtdrehwinkel des Antriebsrotors beim einstellen der Einstechtiefe |
| $\alpha$1, $\alpha$2 | Teildrehwinkel |
| $\beta$ | Drehwinkel |

**Patentansprüche**

1. Mikrosampler-Stechsystem zur Entnahme einer Körperflüssigkeitsprobe aus einem Körperteil, umfassend eine Probenentnahmeeinheit (34, 92, 406) mit einem Stechelement (5, 93), und ein Stechgerät (50, 80) mit

   einem Antrieb (6, 55, 85, 200, 300, 407, 515), mittels dem eine Probenentnahmeeinheit (34, 92, 406) auf einem Bewegungsweg beweglich ist, um das Stechelement (5, 93, 504) mit einer Einstich- und Rückführbewegung in die Haut des Körperteils einzustechen und wieder herauszuziehen,
   einer Einstelleinrichtung (120, 209, 514) zum Einstellen der Einstechtiefe (dm) der zu erzeugenden Einstichwunde, und
einer Steuereinrichtung (1, 90, 214) zum Steuern der Einstich- und Rückführbewegung mit folgenden nacheinander ablaufenden Bewegungsphasen

   - einer Vortriebsphase (V), bei der das Stechelement (5, 93) bis zu einer vorgegebenen Einstechtiefe (dm) eingestochen wird,
   - einer Rückzugsphase (R1), bei der das Stechelement (5, 93, 504) um eine Rückzugsstrecke ($\Delta$d) teilweise zurückgezogen und gegen Ende der Rückzugsphase (R1) abgebremst wird, so daß es mit einer vorgegebenen Reststechtiefe (dr) in die Haut hineinragt und
   - einer Sammelphase (S), in der das Stechelement (5, 93) in die Haut hineinragt und eine Körperflüssigkeitsprobe von der Probenaufnahmeeinheit (34, 92) aufgenommen wird,

   **dadurch gekennzeichnet, daß**
   die Einstelleinrichtung (120, 209, 514) derart ausgebildet ist, daß die Einstechtiefe (dm) unabhängig von dem zeitlichen Mittel der sich während der Sammelphase (S) ergebenden Reststechtiefe (dr) einstellbar ist.

2. Stechsystem nach Anspruch 1, **dadurch gekennzeichnet, daß** das Stechgerät (50, 80) eine Rückführsperre (4, 66, 110, 207) umfaßt, von der das Stechelement (5, 93) am Ende der Rückzugsphase (R1) abgestoppt wird.

3. Stechsystem nach Anspruch 2, **dadurch gekennzeichnet, daß** die Rückführsperre (66) ein Federelement (49) umfaßt, mit dem ein Sperrelement (67) der Rückführsperre (66) zum Abstoppen des Stechelements (5) in eine Blockadeposition gebracht wird.

4. Stechsystem nach einem der vorhergehenden Ansprüche **dadurch gekennzeichnet, daß** ein relativ zu dem Stechelement (5) und relativ zu einem Gerätegehäuse bewegliches Referenzelement (35, 510) bei einem Einstich an der Hautoberfläche anliegt, um eine Einstichwunde mit reproduzierbarer Einstechtiefe zu erzeugen.

5. Stechsystem nach Anspruch 4, **dadurch gekennzeichnet, daß** das Stechelement (5, 504) zumindest während eines Teils der Vortriebsphase (V) zusammen mit dem Referenzelement (35, 510) vorgeschoben wird und das Referenzelement (35, 510) zumindest während eines Teils der Rückzugsphase (R1) zurückgezogen wird.

6. Stechsystem nach Anspruch 5, **dadurch gekennzeichnet, daß** das Referenzelement (35,510) am Ende der Vortriebsphase (V) zusammen mit dem Stechelement (5, 504) vorgeschoben wird, und daß die Strecke, um die das Referenzelement 35,510 gleichzeitig mit dem Stechelement (5,504) vorgeschoben wird, höchstens 5 mm, vorzugsweise höchstens 3,5 mm und besonders bevorzugt höchstens 2 mm beträgt.

7. Stechsystem nach Anspruch 6, **dadurch gekennzeichnet, daß** das Referenzelement (35,510) während eines Teils der Vortriebsphase in einer Ruheposition ruht und ausgehend von der Ruheposition am Ende der Vortriebsphase in Einstichrichtung beschleunigt wird.

8. Stechsystem nach Anspruch 5, **dadurch gekennzeichnet, daß** der Teilabschnitt der Vortriebsphase (V), während dessen das Referenzelement (35,510) gemeinsam mit dem Stechelement (5,504) bewegt wird, maximal 100 msec, bevorzugt maximal 50 msec und besonders bevorzugt maximal 10 msec dauert.

9. Stechsystem nach einem der vorhergehenden Ansprüche, **dadurch gekennzeichnet, daß** die Rückzugsphase (R1) zwischen dem Ende der Vortriebsphase (V) und dem Beginn der Sammelphase (S) höchstens 2 sec, vorzugsweise höchstens 1 sec und besonders bevorzugt 0,5 sec dauert.

10. Stechsystem nach einem der Ansprüche 4 bis 9, **dadurch gekennzeichnet, daß** eine Antriebsfeder (21) während mindestens eines Teils der Vortriebsphase mit dem Stechelement (5) gekoppelt ist und das Referenzelement (35) während mindestens eines Teils desjenigen Teils der Vortriebsphase, in dem die Antriebsfeder (21) mit dem Stechelement (5) gekoppelt ist, über eine Mitnehmereinrichtung mit dem Stechelement (5) und **dadurch** mit der Antriebsfeder (21) gekoppelt ist, wobei die Mitnehmereinrichtung je einen mit dem Stechelement (5) bzw. dem Referenzelement (35) gekoppelten Stechtiefenbegrenzungsanschlag (24,25) aufweist, und die Stechtiefenbegrenzungsanschläge (24,25) derartig aneinander anliegen, daß ihr relativer Abstand in Längsrichtung am Umkehrpunkt der Stechbewegung die Längsposition des Stechelementes (5) relativ zu dem Referenzelement (35) und damit die Stechtiefe definiert.

11. Stechsystem nach Anspruch 10, **dadurch gekennzeichnet, daß** das Referenzelement (10) über eine bidirektional wirkende Mitnehmereinrichtung mit dem Stechelement (5) und **dadurch** mit der Antriebsfeder (21) gekoppelt ist, wobei die Mitnehmereinrichtung je einen mit dem Stechelement (5) bzw. dem Referenzelement (35) gekoppelten in der Rückzugsphase wirkenden Anschlag (43,44) aufweist, und die Anschläge (43,44) während mindestens eines Teils der Rückzugsphase derartig aneinander anliegen, daß **dadurch** die Position des Stechelementes (5) relativ zu dem Referenzelement (35) definiert ist.

12. Stechsystem nach einem der vorhergehenden Ansprüche, **dadurch gekennzeichnet, daß** die Position der Haut vor dem Auslösen einer Einstechbewegung detektiert und die nachfolgende Einstechbewegung an die detektierte Position angepaßt wird.

13. Stechsystem nach einem der vorhergehenden Ansprüche, **dadurch gekennzeichnet, daß** der Antrieb (6, 55, 85, 200, 300, 515) ein Federantrieb ist.

14. Stechsystem nach Anspruch 13, **dadurch gekennzeichnet, daß** der Federantrieb (85, 200, 300) einen von einer Antriebsfeder (86, 203, 302, 501) angetriebenen Antriebsrotor (89, 202, 304, 503) umfaßt.

15. Stechsystem nach einem der vorhergehenden Ansprüche, **dadurch gekennzeichnet, daß** die Steuereinrichtung (1, 90, 214) eine Kurvensteuerung mit einer Steuerkurve (11, 100, 101, 204, 305, 505, 512, 527) und einem Steuerkurvenreiter (12, 102, 103, 205, 306, 506, 526) zum Abfahren der Steuerkurve aufweist.

16. Stechsystem nach Anspruch 15, **dadurch gekennzeichnet, daß** die Steuerkurve (11, 100, 204, 305) einen aktiven Abschnitt, der während der Vortriebsphase (V) und der Rückzugsphase (R1) von dem Steuerkurvenreiter abgefahren wird, aufweist und mindestens eine Grenze des aktiven Abschnitts zum Einstellen der Einstechtiefe (dm) und/oder der Reststechtiefe (dr) verstellbar ist.

17. Stechsystem nach Anspruch 16, **dadurch gekennzeichnet, daß** die, bezogen auf die Bewegung des Steuerkurvenreiters, hintere Grenze des aktiven Abschnitts der Steuerkurve (11, 100, 204, 305) einstellbar ist, um die Reststechtiefe (dr) vorzugeben.

18. Stechsystem nach Anspruch 16 oder 17, **dadurch gekennzeichnet, daß** die, bezogen auf die Bewegung des Steuerkurvenreiters, vordere Grenze des aktiven Abschnitts der Steuerkurve (11, 100, 204, 305) einstellbar ist, um die bei der Vortriebsphase (V) erzielte Einstechtiefe (dm) vorzugeben.

19. Stechsystem nach einem der Ansprüche 15 bis 18, **dadurch gekennzeichnet, daß** die Steuereinrichtung (90) zusätzlich zu der ersten Steuerkurve (110, 505) eine zweite Steuerkurve (101, 512, 527) mit einem zweitem Steuerkurvenreiter (103, 513, 526) zum Abfahren der zweiten Steuerkurve (101, 512, 527) umfaßt.

**20.** Stechsystem nach Anspruch 19, **dadurch gekennzeichnet, daß** die erste Steuerkurve (100, 505) auf einem ersten Teil (111, 503a) des Antriebsrotors (89, 503) und die zweite Steuerkurve (101, 512, 527) auf einem zweiten Teil (112, 503b) des Antriebsrotors (89, 503) angeordnet ist, wobei mittels der Einstelleinrichtung (120, 514) der Abstand zwischen dem ersten Teil (111, 503a) des Antriebsrotors (89, 503) und dem zweiten Teil (112, 503b) des Antriebsrotors (89, 503) in Einstichrichtung variiert werden kann.

**21.** Stechsystem nach Anspruch 19 oder 20, **dadurch gekennzeichnet, daß** die Steuereinrichtung (90) derart ausgebildet ist, daß mindestens einer der beiden Steuerkurvenreiter (102, 103, 506, 526) nur während eines Teils der Einstich- und Rückführbewegung von seiner zugeordneten Steuerkurve (100, 101, 505, 527) geführt wird, vorzugsweise sich in einem mittels der Einstelleinrichtung (120, 514) einstellbaren Bewegungsabschnitt der Einstich- und Rückführbewegung von seiner zugeordneten Steuerkurve (100, 101, 505, 527) löst.

**22.** Stechsystem nach einem der vorhergehenden Ansprüche, **dadurch gekennzeichnet, daß** die Steuereinrichtung (1) ein Schwenkelement (2) umfaßt, das aus einer Anfangsposition in eine Endposition schwenkbar ist und dabei eine Einstich- und Rückführbewegung des Stechelements (5) bewirkt, wobei die Endposition durch einen Endanschlag (4) vorgegeben ist, der zum Einstellen der am Ende der Rückzugsphase (R1) erzielten Reststechtiefe (dr) verschieblich ist.

**23.** Stechsystem nach Anspruch 22, **dadurch gekennzeichnet, daß** das Schwenkelement (2) eine Parallelogrammführung ist.

**24.** Stechsystem nach Anspruch 22, **dadurch gekennzeichnet, daß** das Schwenkelement (2) ein Kniehebel ist.

**25.** Stechsystem nach einem der Ansprüche 22 bis 24, **dadurch gekennzeichnet, daß** die Anfangsposition durch einen einstellbaren Anfangsanschlag definiert wird.

**26.** Stechsystem nach einem der vorhergehenden Ansprüche, **dadurch gekennzeichnet, daß** die Rückführbewegung im Anschluß an die Sammelphase (S) eine weitere Rückzugsphase (R2) umfaßt, bei der das Stechelement (5) erneut beschleunigt und vollständig aus der Haut herausgezogen wird.

**27.** Stechsystem nach einem der vorhergehenden Ansprüche, **dadurch gekennzeichnet; daß** die Einstelleinrichtung (120, 209) derart ausgebildet ist, daß die Reststechtiefe (dr) unabhängig von der Einstechtiefe (dm) einstellbar ist.

**28.** Stechgerät, insbesondere für ein Stechsystem nach einem der vorhergehenden Ansprüche, umfassend einen Lanzettenträger (405) zur Aufnahme einer Probenentnahmeeinheit (406) und einen Antrieb (407) zum Beschleunigen des Lanzettenträgers (405) für eine Einstich- und Rückführbewegung, **dadurch gekennzeichnet, daß** der Lanzettenträger (405) mit einem Dämpfungsmechanismus (315, 400) gekoppelt ist, um das Abbremsen des Lanzettenträgers (405) gegen Ende einer Rückführbewegung zu dämpfen.

**29.** Stechgerät nach Anspruch 28, **dadurch gekennzeichnet, daß** der Dämpfungsmechanismus (315, 400) einen Rotationsdämpfer (316, 401) enthält.

**30.** Stechgerät nach Anspruch 29, **dadurch gekennzeichnet, daß** der Rotationsdämpfer (316, 401) eine Kammer aufweist, in der ein Rotationskörper (313, 402) in einer Dämpfungsflüssigkeit drehbar gelagert ist.

Fig.1

Fig.2

Fig.3

20

Fig.4

Fig.5

Fig.6

Fig.7

Fig. 8

Fig. 11

Fig. 9

Fig. 12

Fig. 10

Fig. 13

Fig.14

Fig.15

Fig. 16

Fig. 17

Fig 18

Fig. 19

Fig. 20

Fig. 21

Fig.22

Fig.23

Fig.24

Fig.25

Fig.26

Fig.27

Fig. 28

fig. 29

Fig. 30

Fig. 31

**Europäisches Patentamt**

**EUROPÄISCHER RECHERCHENBERICHT**

Nummer der Anmeldung

EP 05 02 7428

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2004/059256 A1 (PEREZ EDWARD) 25. März 2004 (2004-03-25) * Absätze [0045], [0056], [0065]; Abbildungen 1,2 * ----- | 1-3 | INV. A61B5/15 A61B10/00 |
| X | WO 02/100275 A (AMIRA MEDICAL, INC; ROE, JEFFREY, N; ALLEN, JOHN, J) 19. Dezember 2002 (2002-12-19) * Seite 11, Zeilen 19-29 * * Seite 12, Zeilen 9-17 * ----- | 1-3 | |
| D,A | US 2003/018282 A1 (EFFENHAUSER CARLO ET AL) 23. Januar 2003 (2003-01-23) * Absatz [0023] * ----- | 1 | |
| A | US 4 637 403 A (GARCIA ET AL) 20. Januar 1987 (1987-01-20) * Spalte 12, Zeilen 15-25; Abbildungen 11,12 * ----- | 1 | |
| A | WO 2004/041087 A (LIFESCAN, INC; ALLEN, JOHN, J) 21. Mai 2004 (2004-05-21) * Anspruch 1; Abbildung 3J * ----- | 1 | RECHERCHIERTE SACHGEBIETE (IPC) A61B A61M |
| A | US 2002/103499 A1 (PEREZ EDWARD P ET AL) 1. August 2002 (2002-08-01) * Absätze [0087], [0088], [0091]; Anspruch 1 * ----- | 1 | |

~~Der vorliegende Recherchenbericht wurde für alle Patentans~~prüche erstellt

| Recherchenort | Abschlußdatum der Recherche | Prüfer |
|---|---|---|
| München | 30. März 2006 | Anscombe, M |

EPO FORM 1503 03.82 (P04C03)

**Europäisches**
**Patentamt**

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

1-3

**Europäisches**
**Patentamt**

**MANGELNDE EINHEITLICHKEIT**
**DER ERFINDUNG**
**ERGÄNZUNGSBLATT B**

Nummer der Anmeldung

EP 05 02 7428

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-3

        Stechsystem mit Rückführsperre
         ---

2. Ansprüche: 4-8,10-12

        Stechsystem mit beweglichem Referenzelement
         ---

3. Ansprüche: 13,14

        Stechsystem mit Federantrieb
         ---

4. Ansprüche: 15-21

        Stechsystem mit Kurvensteuerung
         ---

5. Ansprüche: 22-25

      Stechsystem mit Schwenkelement mit einstellbarer Endanschlag
         ---

6. Ansprüche: 26,9

        Stechsystem mit bis zu zwei Rückführbewegungen
         ---

7. Anspruch: 27

        Stechsystem mit einstellbarer Reststechtiefe
         ---

8. Ansprüche: 28-30

        Stechgerät mit Lanzettenträgerantrieb
         ---

**ANHANG ZUM EUROPÄISCHEN RECHERCHENBERICHT
ÜBER DIE EUROPÄISCHE PATENTANMELDUNG NR.**

EP 05 02 7428

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-03-2006

| Im Recherchenbericht angeführtes Patentdokument | Datum der Veröffentlichung | Mitglied(er) der Patentfamilie | Datum der Veröffentlichung |
|---|---|---|---|
| US 2004059256 A1 | 25-03-2004 | KEINE | |
| WO 02100275 A | 19-12-2002 | CA 2450106 A1 | 19-12-2002 |
| | | EP 1416852 A1 | 12-05-2004 |
| | | JP 2004532695 T | 28-10-2004 |
| US 2003018282 A1 | 23-01-2003 | DE 10134650 A1 | 06-02-2003 |
| | | WO 03009759 A1 | 06-02-2003 |
| | | EP 1420694 A1 | 26-05-2004 |
| US 4637403 A | 20-01-1987 | AU 5699086 A | 05-11-1986 |
| | | DE 3687994 D1 | 22-04-1993 |
| | | EP 0199484 A2 | 29-10-1986 |
| | | WO 8605966 A1 | 23-10-1986 |
| WO 2004041087 A | 21-05-2004 | AU 2003285103 A1 | 07-06-2004 |
| | | AU 2003286783 A1 | 07-06-2004 |
| | | CA 2473661 A1 | 21-05-2004 |
| | | CA 2473670 A1 | 21-05-2004 |
| | | CN 1684628 A | 19-10-2005 |
| | | CN 1691917 A | 02-11-2005 |
| | | EP 1558139 A2 | 03-08-2005 |
| | | EP 1558140 A1 | 03-08-2005 |
| | | JP 2006512110 T | 13-04-2006 |
| | | JP 2006512111 T | 13-04-2006 |
| | | MX PA04006476 A | 13-07-2005 |
| | | MX PA04006477 A | 13-07-2005 |
| | | WO 2004041088 A1 | 21-05-2004 |
| US 2002103499 A1 | 01-08-2002 | US 2005004494 A1 | 06-01-2005 |

EPO FORM P0461

Für nähere Einzelheiten zu diesem Anhang : siehe Amtsblatt des Europäischen Patentamts, Nr.12/82

**EP 1 797 822 A1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 03009759 A1
- US 20030018282 A1
- WO 0189383 A2